# EUROPEAN PATENT APPLICATION

(11) **EP 1 900 747 A1**
(43) Date of publication of application: **19.03.2008**
(21) Application number: 06746761.3
(22) Date of filing: 23.05.2006
(51) Int. Cl.: C07K 5/078, C07K 5/097, C07K 5/117, H01M 14/00, H01L 31/04

(54) **COMPOUND HAVING PHOTOSENSITIZATION ACTIVITY, PHOTOELECTRODE, AND PHOTOSENSITIZATION-TYPE SOLAR CELL**

(30) Priority: 23.05.2005 JP 2005148880
(71) Applicant: Credia Japan Co., Ltd., Kamigyo-ku Kyoto-shi Kyoto 6020841 (JP)
(72) Inventor: IKEDA, Hisafumi, Ukyo-ku, Kyoto-shi, Kyoto 6150066 (JP); TONOSAKI, Madoka c/o CREDIA JAPAN CO., LTD., Mikurumamichi Kyoto-shi Kyoto (JP)
(74) Representative: HOFFMANN EITLE
(86) International application number: PCT/JP2006/310277
(87) International publication number: WO 2006/126557

(57) **Abstract**

The main object of the invention is to provide a compound that can be used as a sensitizing dye of a dye-sensitized solar cell, which can realize outstanding endurance and photovoltaic conversion efficiency.
1. A compound shown by the following general formula (1) is therefore provided: 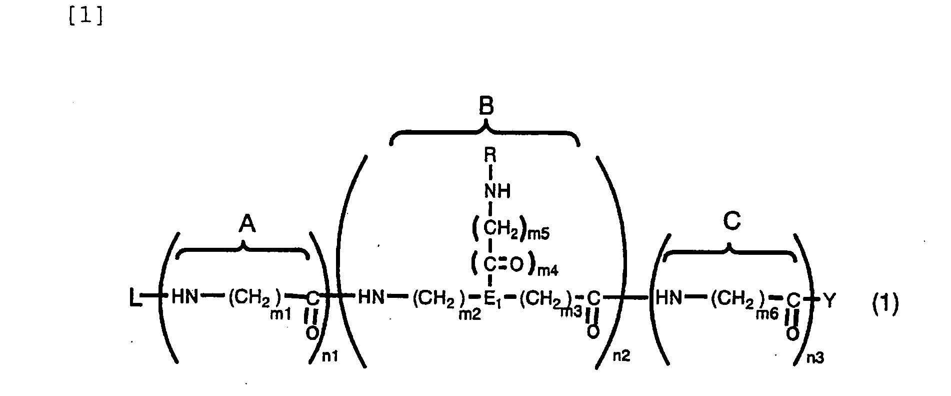 (wherein, in the formula (1), Y is a hydroxyl group or an amino group; E1 is N or CH; L is a hydrogen atom or a photosensitizing group; R is a group having one of the basic units D shown by the following general formula (D), or a group having a branched structure wherein a structural unit is repeatedly bonded via a peptide linkage, this structural unit being 1-100 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms).

## Description

### TECHNICAL FIELD

The present invention relates to a compound that has a photosensitizing effect, and is useful in a photosensitized solar cell. The invention further relates to a method of manufacturing this compound, to a photoelectrode using said compound, to a photosensitized solar cell, and to a photosensitized solar cell module.

### BACKGROUND ART

In the field of photochemical energy, dye-sensitized solar cells are attracting worldwide attention as a leading candidate for next-generation voltaic cells. In this dye-sensitized solar cell, when the sensitizing dye adsorbed on a semiconductor electrode is exposed to light, the electrons in this sensitizing dye are excited, and the excited electrons are implanted in the semiconductor electrode.
Photovoltaic conversion is performed by producing a current from the electrons generated by this semiconductor electrode, and extracting electrical energy.

To make dye-sensitized solar cells commercially viable, higher endurance and photovoltaic conversion efficiency are required. In a conventional dye-sensitized solar cell, there is a problem that electrons which have been excited cannot be extracted from the sensitizing dye effectively.
Further, in a conventional dye-sensitized solar cell, there is a problem that the sensitizing dye adsorbed on the semiconductor electrode is desorbed from this semiconductor electrode, and as a result, photovoltaic conversion efficiency falls with passage of time.

Therefore, in the field of dye-sensitized solar cells, it has been desired to develop a compound from which electrons excited by optical exposure can be efficiently extracted, and which is not easily desorbed from a semiconductor electrode.

### DISCLOSURE OF THE INVENTION

### PROBLEMS TO BE SOLVED BY THE INVENTION

It is therefore an object of the invention to provide a compound that can be used as a sensitizing dye of a dye-sensitized solar cell, and which has outstanding endurance and photovoltaic conversion efficiency. Specifically, it is an object of the invention to provide a compound from which electrons excited by optical exposure can be efficiently extracted, and which is not easily desorbed from a semiconductor electrode. It is another object of the invention to provide a method of manufacturing said compound. It is a still further object of the invention to provide a photoelectrode, a photosensitized solar cell, and a photosensitized solar cell module using said compound.

### MEANS FOR SOLVING THE PROBLEMS

The inventors, as a result of intensive efforts made to solve this problem, discovered that in the newly synthesized compound shown by the general formula (1), electrons excited by photoexposure could be efficiently extracted, and also found that the compound was not easily desorbed from a semiconductor electrode, and was useful as a sensitizing dye of a dye-sensitized solar cell.
The invention has been accomplished by conducting intensive research based on this observation.

Specifically, the invention provides the compound given below, its manufacturing method, a photoelectrode, a photosensitized solar cell, and a photosensitized solar cell module.
Item 1. Compound shown by the following general formula (1):

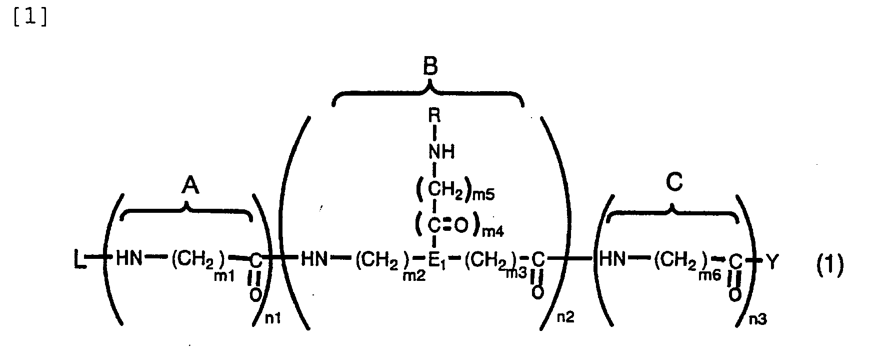

(wherein n1 is an integer of 0-10, n2 is an integer of 1-50, and n3 is an integer of 1-10; m1 is an integer of 0-100, m2 is an integer of 0-100, m3 is an integer of 0-100, m4 is an integer equal to 0 or 1, m5 is an integer of 0-100, and m6 is an integer of 0-100; Y is a hydroxyl group or an amino group; E1 is N or CH; L is a hydrogen atom or a photosensitizing group; R is a group consisting of one of the basic units D shown by the following general formula (D), or a group having a branched structure wherein a structural unit is repeatedly bonded via a peptide linkage, this structural unit being 1-100 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms. R is bonded via a peptide linkage to a nitrogen atom in the repeating unit B; in R, when two or more of the basic units D are bonded together, these basic units may be identical or different; in R, when two or more of the basic units G are bonded together, these basic units may be identical or different; when n1 is 2 or more, m1 in the repeating unit A may be identical or different in respective repeating units A; when n2 is 2 or more, m2-m5 and R in the repeating unit B may be identical or different in respective repeating units B; and when n3 is 2 or more, m6 in the repeating unit C may be identical or different in respective repeating units C).

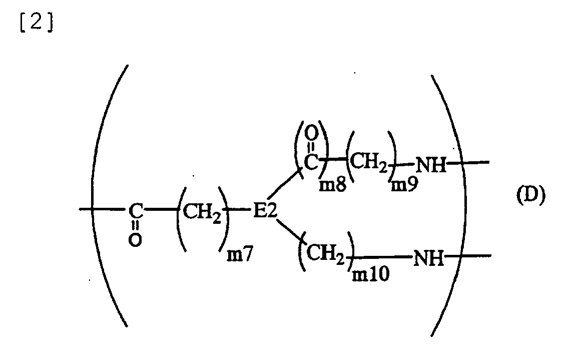

(wherein, in the formula (D), m7 is an integer of 0-100, m8 is an integer equal to 0 or 1, m9 is an integer of 1-100, and m10 is an integer of 0-100; and E2 is N or CH).

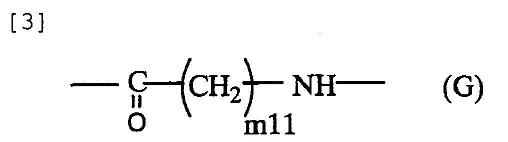

(wherein, in the formula (G), m11 is an integer of 1-100). Item 2. The compound according to Item 1, wherein R is a group including 1-100 of the basic units D, wherein m7 is 1 or 2, m8 is 1, m9 is an integer of 0-11, m10 is an integer of 1-3, and E2 is N. Item 3. The compound according to Item 1, wherein R is a group including 1-100 of the basic units D, wherein m7 is 0-2, m8 is 0, m9 is an integer of 0-11, and m10 is 0-2, and E2 is CH.
Item 4. The compound according to Item 1, wherein n1 is an integer of 0-5, n2 is an integer of 1-20, and n3 is an integer of 0-5.
Item 5. The compound according to Item 1, wherein n1 is an integer of 0-2, n2 is an integer of 1-10, and n3 is an integer of 0-2.
Item 6. A method of manufacturing the compound shown by the following general formula (1), including the following steps 1-1 to 1-6.

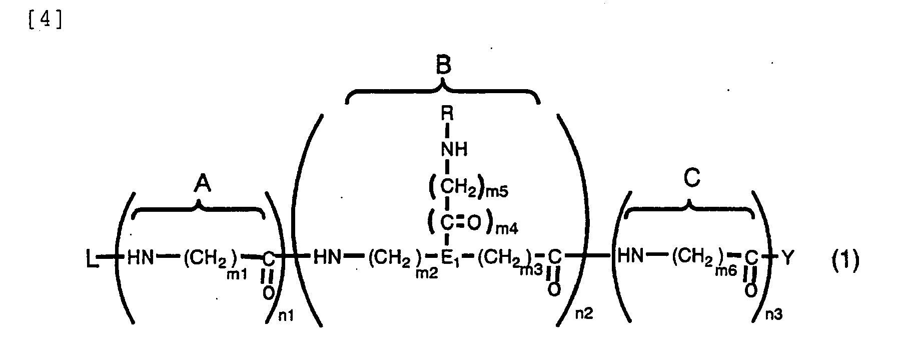

In the formula (1), n1 is an integer of 0-10, n2 is an integer of 1-50, and n3 is an integer of 1-10; m1 is an integer of 0-100, m2 is an integer of 0-100, m3 is an integer of 0-100, m4 is an integer equal to 0 or 1, m5 is an integer of 0-100, m6 is an integer of 0-100; Y is a hydroxyl group or an amino group; E1 is N or CH; L is a hydrogen atom or a photosensitizing group; R is a group having one of the basic units D shown by the following general formula (D), or a group having a branched structure wherein a structural unit is repeatedly bonded via a peptide linkage, this structural unit being 1-10 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms. R is bonded via a peptide linkage to a nitrogen atom in the repeating unit B; in R, when two or more of the basic units D are bonded together, these basic units may be identical or different; in R, when two or more of the basic units G are bonded together, these basic units may be identical or different; when n1 is 2 or more, m1 in the repeating unit A may be identical or different in respective repeating units A; when n2 is 2 or more, m2-m5 and R in the repeating unit B may be identical or different in respective repeating units B; and when n3 is 2 or more, m6 in the repeating unit C may be identical or different in respective repeating units C.

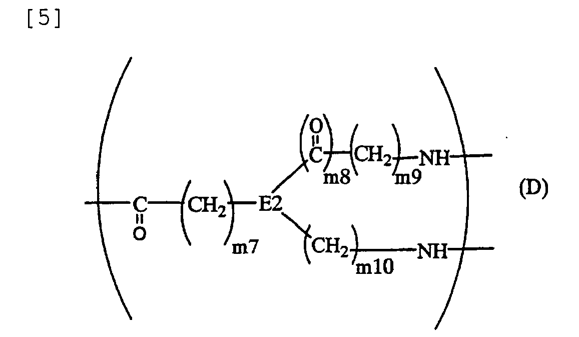

(wherein, in the formula (D), m7 is an integer of 0-100, m8 is an integer equal to 0 or 1, m9 is an integer of 1-100, m10 is an integer of 0-100; and E2 is N or CH).

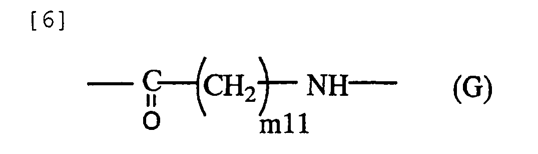

(wherein, in the formula (G), m11 is an integer of 1-100). Step 1-1: The compound shown by the general formula (i):

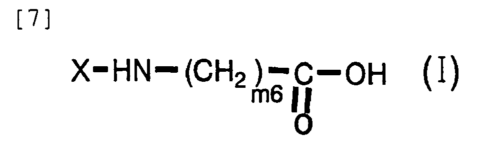

(wherein, in the formula (I), m6 is identical to the above, and X is a protective group),
is obtained by performing a condensation of the compound shown by the general formula (I) with a solid phase resin or a solid phase compound, removing the protective group X of the compound that was condensed with the solid phase resin or solid phase compound, and performing a condensation polymerization of the compound shown by the general formula (I), n3-1 times.

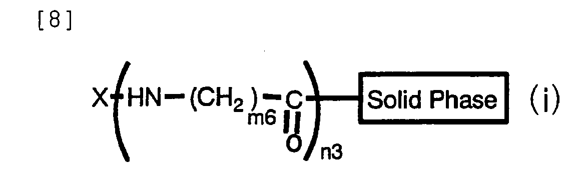

(wherein, in the formula (i), n3, m6, and X are identical to the above, and Solid Phase denotes a solid phase resin or a solid phase compound).
Step 1-2: The compound shown by the general formula (ii) is obtained by performing a condensation reaction of the compound shown by the general formula (II) with the compound shown by the general formula (i), n2 times.

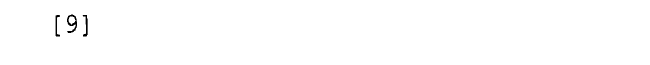

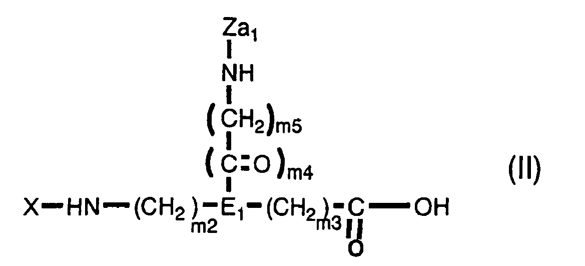

(wherein, in the formula (II), m2-m5, E1 and X are identical to the above, and Za1 is a protective group different from X).

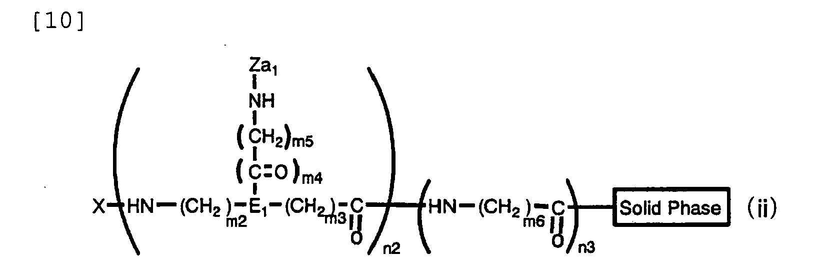

(wherein, in the formula (ii), n2, n3, m2-m6, X, E1, Za1 and Solid Phase are identical to the above).
Step 1-3: The compound shown by the general, formula (iii) is obtained by performing a condensation reaction of the compound shown by the general formula (III) with the compound shown by the general formula (ii), n1 times.

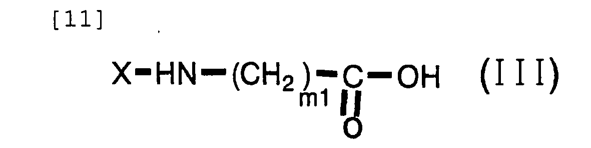

(wherein, in the formula (III), ml and X are identical to the above).

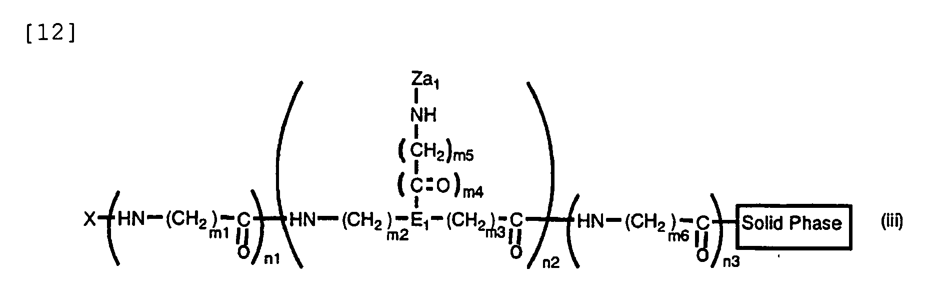

(wherein, in the formula (iii), n1-n3, m1-m6, X, E1, Za1 and Solid Phase are identical to the above).
Step 1-4: The compound shown by the general formula (iv) is obtained by performing a condensation reaction of one of the compounds shown by the following general formula (IV) with the compound shown by the general formula (iii).

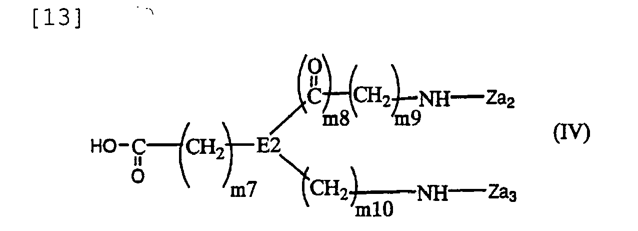

(wherein, in the formula (IV), m7-m10 are identical to the above, Za2 and Za3 are protective groups which may be respectively identical or different, which are different from X, but which may be identical to Za1).
Alternatively, the compound shown by the following general formula (iv) is obtained by performing a condensation reaction of 1-100 of the compounds shown by the general formula (IV) and 1-100 compounds shown by the general formula (V) with the compound shown by the general formula (iii).

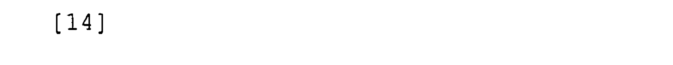

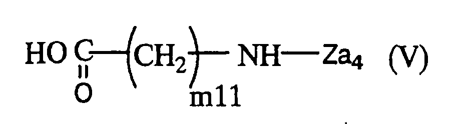

(wherein, in the formula (V), m11 is identical to the above, Za4 is a protective group different from X, and may be identical to Za1, Za2, and Za3).

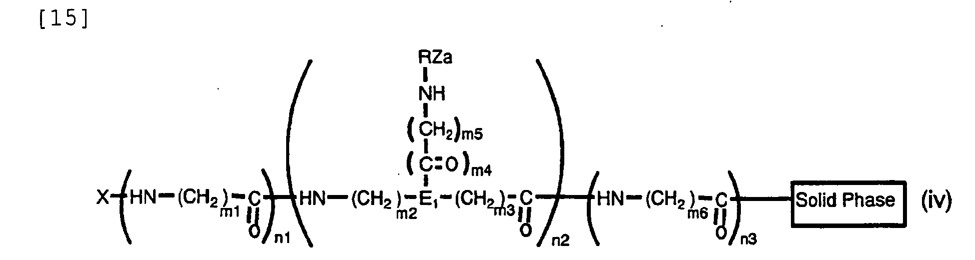

(wherein, in the formula (iv), n1-n3, m1-m6, X, and Solid Phase are identical to the above. Rza is a group consisting of one of the basic units D shown by the following general formula (D), or a group having a branched structure wherein a structural unit is repeatedly bonded via a peptide linkage, this structural unit being 1-100 of the basic units D and 1-100 of the basic units G shown by the following general formula (G), and denotes a group wherein the nitrogen atoms which do not constitute the peptide linkage of this group are protected by the protective groups Za3 and Za4, or the protective groups Za2, Za3, and Za4).
Step 1-5: One, two or more of the protective groups Za2, Za3, Za4, and X are removed from the compound shown by the general formula (iv), and a photosensitizing group is bonded to the nitrogen atoms to which this protective group was bonded.
Step 1-6: The solid phase resin or solid phase compound is separated from the compound obtained in the above step 1-5, a hydroxyl group or an amino group is bonded to the carbon atom with which this solid phase resin or solid phase compound was bonded, the protective groups which were not removed in the step 1-5 are removed, a hydrogen atom is bonded to the nitrogen atom to which this protective group was bonded, and the compound shown by the general formula (1) is thereby obtained.
Item 7. A photoelectrode comprising a conductive substrate, and a porous semiconductor layer formed on this substrate on which the compound shown by the general formula (1) is adsorbed.
Item 8. A dye-sensitized solar cell comprising the photoelectrode according to Item 7 and a counter-electrode that opposes to the photoelectrode with an electrolyte layer therebetween.
Item 9. A dye-sensitized solar cell module having two or more of the dye-sensitized solar cells according to Item 8 connected in series as a unit cell.

### EFFECTS OF THE INVENTION

Electrons excited by optical exposure of the compound of the invention can be efficiently extracted, and since the compound is not easily desorbed from a semiconductor electrode, it is useful as a sensitizing dye of a dye-sensitized solar cell.

Moreover, the photoelectrode of the invention has outstanding endurance and photovoltaic conversion efficiency, which is very useful when used in a photosensitized solar cell and a photosensitized solar cell module.

### DETAILED DESCRIPTION OF THE PREFERRED EMBODIMENTS

### 1. Compound shown by the general formula (1)

The invention provides the compound shown by the general formula (1) having the following structure.

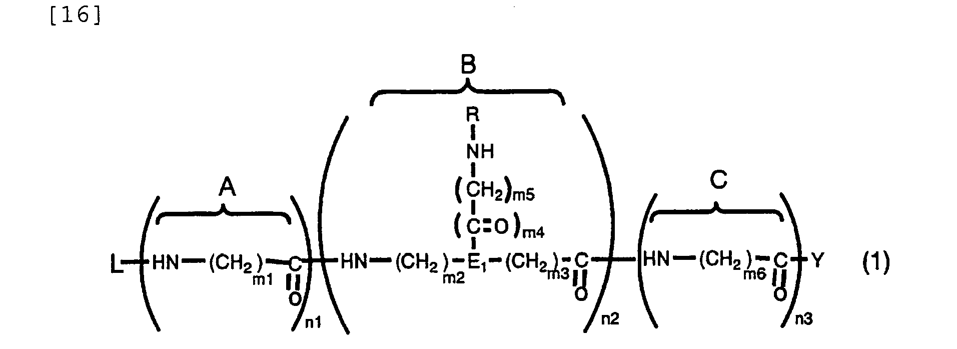

In the formula (1), n1 shows the number of repeating units A, and is an integer of 0-10, preferably an integer of 0-5, and more preferably an integer of 0-2.

In the repeating unit A, m1 is an integer of 0-100, preferably an integer of 0-30, and more preferably an integer of 0-11. If n1 is an integer equal to 2 or more, i.e., if there are 2 or more of the repeating units A, m1 may be identical or different in the repeating units A.

In the formula (1), n2 is the number of repeating units B, and is an integer of 0-50, preferably an integer of 0-20, and more preferably an integer of 1-10.

In the repeating unit B, m2 is an integer of 0-100, preferably an integer of 0-20, and more preferably an integer of 0-2. m3 is an integer of 0-100, preferably an integer of 0-20, and more preferably an integer of 0-2. m4 is an integer equal to 0 or 1. m5 is an integer of 0-100, preferably an integer of 0-30, and more preferably an integer of 0-11.

In the repeating unit B, E1 is N or CH. In the repeating unit B, if E1 is N, m2 is 0-2 and preferably 2; m3 is 0-1, and preferably 1; m4 is 1, m5 is 0-11, and preferably 5. In the repeating unit B, if E1 is CH, m2 is 0-2 and preferably 0; m3 is 0-1, and preferably 0; m4 is 0; and m5 is 0-11, and preferably 4.

In the repeating unit B, R may be either the group shown by the following (a) or (b), but is preferably the group shown by (b):
(a) a group consisting of one of the basic units D shown by the following general formula (D), wherein a photosensitizing group is bonded to one or both of the nitrogen atoms at the terminal of this basic unit D.
(b) a group having a branched structure wherein a structural unit is repeatedly bonded via a peptide linkage, the structural unit being 1-10 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms.
In the invention, a peptide linkage is synonymous with an amide bond, -(NH-CO)-.

R is bonded to the nitrogen atom in the repeating unit B via a peptide linkage.

In the above group (a), a photosensitizing group is preferably bonded to both of the two nitrogen atoms at the terminal of this basic unit D.

In the above group (b), the group preferably contains 1-7, and more preferably 1-3 of the basic units D; and preferably contains 1-30, more preferably 1-10 of the basic units G.

In the group (b), the manner in which the basic unit D and basic unit G are bonded is not particularly limited, but they may be bonded such that the basic unit D and basic unit G alternate regularly with each other, or the basic unit D and the basic unit G may be bonded with each other in any order. The basic unit D contains two nitrogen atoms that can form a peptide linkage. The basic unit D or the basic unit G may be bonded to either thereof, or, the basic unit D or the basic unit G may be bonded to both thereof. Further, among the two nitrogen atoms which can form the peptide linkage in the basic unit D, the basic unit D may be bonded to one thereof, and the basic unit G bonded to the other thereof.

As an preferable example of the group (b), the group (b1) shown in the following formula has three basic units D and six basic units G, a basic unit D, a basic unit G, a basic unit D, and a basic unit G that are bonded in this order.

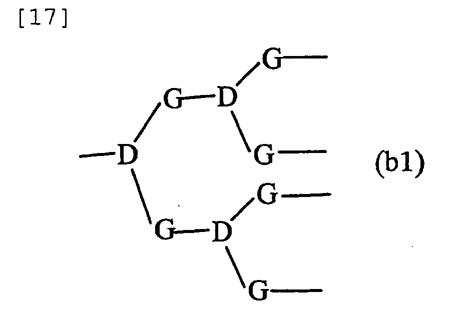

(wherein, in the formula (b1), D is a basic unit D represented by the general formula (D), and E is a basic unit (G) represented by the general formula (E)).
In the group (b), among the nitrogen atoms which do not form the peptide linkage, the photosensitizing group may be bonded to 50% or more, preferably 75% or more, and more preferably 90% or more thereof, but it is particularly preferably bonded to all of them. The larger the number of photosensitizing groups is, the higher is the photovoltaic conversion efficiency of the compound that can be provided.

Specifically, the basic unit D has the following structure.

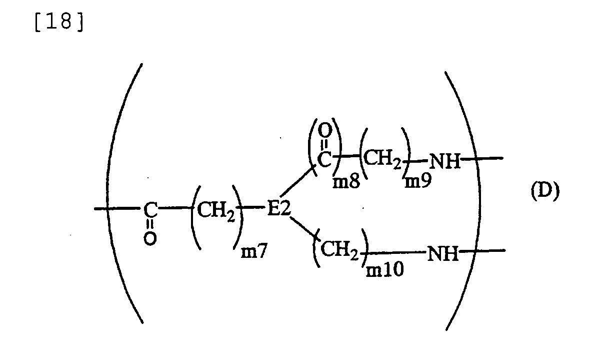

In the formula (D), m7 is an integer of 0-100, preferably an integer of 0-20 and more preferably an integer of 0-2.
M8 is an integer equal to 0 or 1, M9 is an integer of 0-100, preferably an integer of 0-30, and more preferably an integer of 0-11. M10 is an integer of 0-100, preferably an integer of 0-20, and more preferably an integer of 0-2.

In the formula (D), E2 is N or CH. In the basic unit D, when E2 is N, m7 is 1 or 2, and preferably 1; m8 is 1; m9 is 0-11, and preferably 5; m10 is 1-3, and preferably 2. In the basic unit D, when E2 is CH, m7 is 0-2 and preferably 0; m8 is 0; m9 is 0-11, and preferably 4; and m10 is 0-2, and preferably 0.

When R contains two or more of the basic units D, the basic units D may be respectively identical or different.

The basic unit G specifically has the following structure.

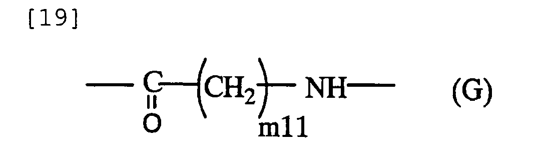

In the formula (D), m11 is an integer of 0-100, preferably an integer of 0-30, and more preferably an integer of 0-11.

When R contains two or more of the basic units G, the basic units G may be respectively identical or different.

The photosensitizing group used in R is a group which has a photosensitizing effect, i.e., a group which can produce excited electrons by sunlight exposure. This photosensitizing group is preferably a compound currently used as a sensitizing dye of a photosensitized solar cell.
Specific examples of the photosensitizing group are a moiety containing a pigment, such as a ruthenium complex, porphyrin, phthalocyanine, coumarin, carbazole, thiophene, chlorophyll, an azo dye, a quinone dye or an indigo dye. In these photosensitizing groups, from the viewpoint that they have an excellent light absorption coefficient and light resistance, groups containing a ruthenium complex dye are preferred.

When the above pigment has a carboxyl group, the residue from which the hydroxyl of the carboxyl group of the pigment has been removed, may be used as the photosensitizing group. A group wherein a suitable linker is bonded to the pigment, and which can bond to a nitrogen atom of the basic unit D and/or the basic unit G, may for example also be used as the photosensitizing group.

Specific examples of the photosensitizing group are the following.

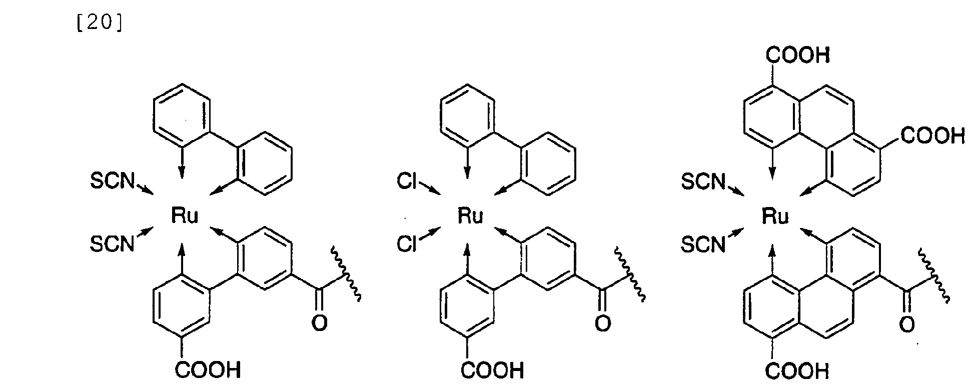

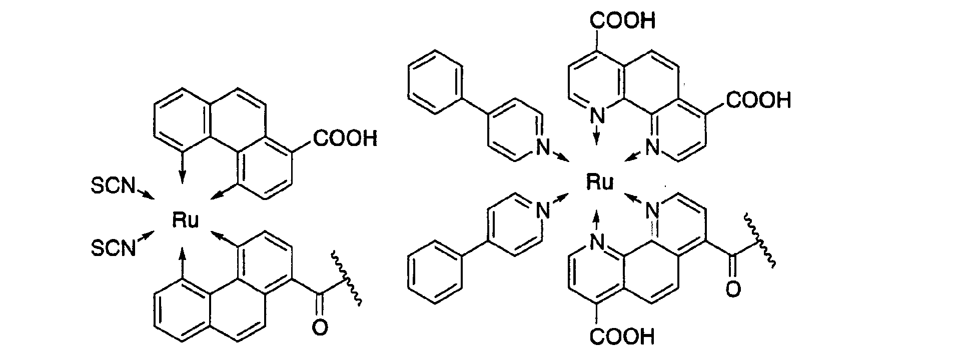

In the invention, the above photosensitizing groups may be used alone, or two or more may be used together.

In the repeating unit B, if m2 is an integer equal to 2 or more, i.e., if there are 2 or more of the repeating units B, m2-m5 and R may be identical or different among the respective repeating units B.

In the formula (1), n3 is the number of repeating units C, and is an integer of 0-10, preferably an integer of 0-5, and more preferably an integer of 0-2.

In the repeating unit C, m6 is an integer of 0-100, preferably an integer of 0-30 and more preferably an integer of 0-11. If n3 is an integer equal to 2 or more, i.e., if there are 2 or more of the repeating units C, m6 may be identical or different among the respective repeating units C.

As specific examples of n1-n3 in the formula (1), n1 is an integer of 0-2, n2 is an integer of 0-10, and n3 is an integer of 0-2, and particularly preferably, n1 is an integer equal to 1 or 2, n2 is an integer of 4-6, and n3 is an integer equal to 1 or 2.

In the formula (1), Y is a hydroxyl group or an amino group.

In the formula (1), L is a hydrogen atom or a photosensitizing group.
The photosensitizing group constituting L may be identical to the photosensitizing group in R of the repeating unit B. L is preferably a photosensitizing group.

### 2. Method of manufacturing the compound shown by the general formula (1)

The compound shown by the general formula (1) may be manufactured by performing the following steps 1-1 to 1-5 in sequence (the first method). The steps 1-1 to 1-5 will now be described in detail.

### Step 1-1

In the step 1-1, the compound shown by the general formula (i) is synthesized using the compound shown by the following general formula (I) and a solid phase resin or a solid phase compound.

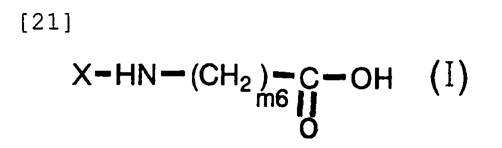

In the formula (I), m6 is identical to the above.

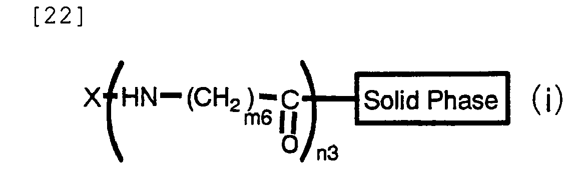

In the formula (i), n3 and m6 are identical to the above, and X is a protective group.
Solid Phase is a solid phase resin or a solid phase compound.

Here, the protective group is a group providing protection so that the bonding groups in a particular region of the compound are not affected by reactions due to oxidation, reduction, hydrolysis, condensation, etc., in other constituent regions of this compound, and denotes a group which can be removed under predetermined conditions to be substituted by a hydrogen atom or hydroxyl group.
A specific example of the protective group is tert-butoxycarbonyl (Boc group) or 9-fluorenyl methoxycarbonyl (Fmoc group), other examples apart from these being shown below.

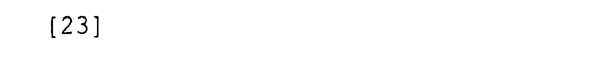

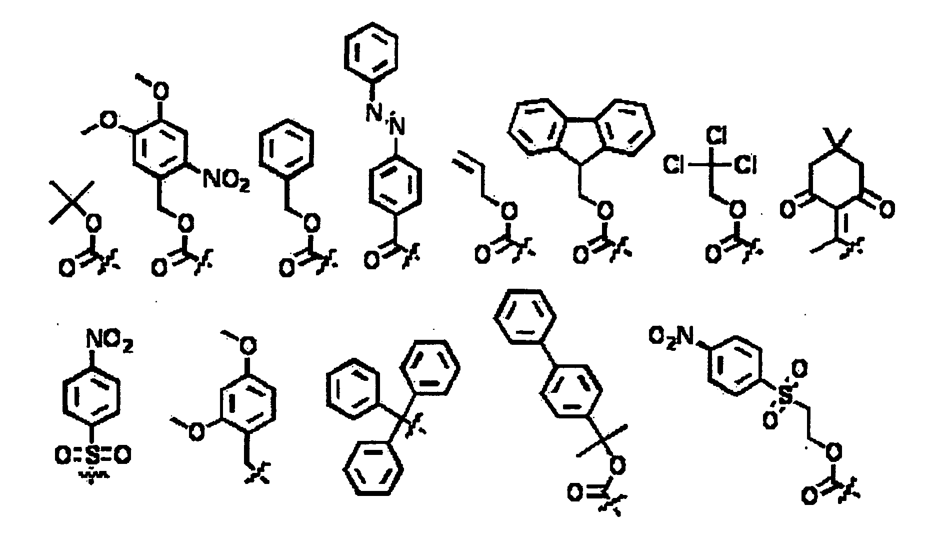

The protective group X is preferably Boc or Fmoc.

The compound shown by the general formula (I) is a publicly known compound or a compound manufactured according to a publicly known manufacturing method.

The solid phase resin or solid phase compound used in this step is not particularly limited, specific examples being MBHA (methylbenzodrylamine resin), PAL (peptide amide linker), Oxime (P-nitrobenzo phenone oxime), PAM (4-hydroxymethylphenylacetamide methyl resin), Merrifield resin and the like.

First, the compound shown by the general formula (I) is made to undergo a condensation reaction with the solid phase resin or solid phase compound.

The condensation reaction of the compound shown by the general formula (I) with the solid phase resin or solid phase compound, is performed by reacting normally 0.01-100 mol and preferably 0.1-10 mol of the solid phase resin or solid phase compound with respect to 1 mol of the compound shown by the general formula (I).

The condensation reaction of the compound shown by the general formula (I) with the solid phase resin or solid phase compound is usually performed in a suitable solvent. Any solvent known in the art may be used for this purpose provided that it does not interfere with the reaction. Examples of such a solvent are dimethylformamide (DMF), N-methylpyrrolidone (NMP), and the like.

The condensation reaction of the compound shown by the general formula (I) with the solid phase resin or solid phase compound is preferably performed using a condensing agent and a reaction accelerator. Examples of the condensing agent are O-(azabenzotriazole-1-yl)-N,N,N',N'-tetramethyluronium hexafluorophosphorate (HATU), O-(benzotriazole-1-yl)-N,N,N',N'-tetramethyluronium hexafluorophosphorate (HBTU), 1-ethyl-3-(3-dimethylaminopropyl) carbodiimide (EDCI) hydrochloride, dicyclohexylcarbodiimide (DCC), etc. As the condensing agent, HATU is preferably used. Examples of the reaction accelerator are N,N-diisopropylethylamine (DIEA), triethylamine (TEA), etc. As the reaction accelerator, DIEA is preferably used.

The usage amount of the condensing agent is normally 0.01-100 mol and preferably 0.1-10 mol in total with respect to 1 mol of the solid phase resin or solid phase compound.

The usage amount of the reaction accelerator is normally 0.01-100 mol and preferably 0.1-10 mol in total with respect to 1 mol of the solid phase resin or solid phase compound.

The condensation reaction of the compound shown by the general formula (I) with the solid phase resin or solid phase compound is normally performed at 5-80°C and preferably 10-30°C, for 0.1-48 hours and preferably 0.1-1 hour, with stirring if required.

In this way, the compound having the following structure wherein one of the compounds shown by the general formula (I) is condensed with the solid phase resin or solid phase compound, can be obtained. Hereafter, when the compound is bonded to the solid phase resin or solid phase compound, it will be referred to as a solid phase-bonded compound.

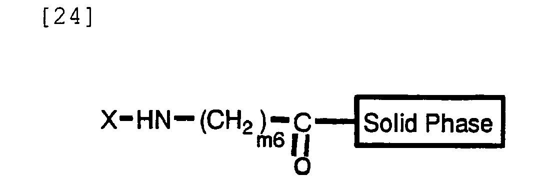

The protective group X is then removed from the solid phase-bonded compound thus obtained.
The removal of the protective group X of the solid phase-bonded compound is performed by a suitable method depending on the nature of the protective group X. For example, when the protective group X is Boc, the method of treating at 10-30°C for 0.1 to 1 hour in a TFA (trifluoroacetic acid) solution (95 vol% TFA/5 vol% m-cresol) may be given. When the protective group X is Fmoc, the method of treating at 10-30°C for 0.01 to 0.5 hour in a piperidine solution (20 vol% piperidine/80 vol% dimethylformamide) may be employed. When the protective group X is Aloc, the method of treating at 10-30°C for 0.1 to 1 hour in a Pd(PPh₃)₄ (tetrakistriphenylphosphine palladium complex, 0.1-10 wt%) solution (55 vol% chloroform/30 vol% acetic acid/15 vol% N-methylmorpholine) may be employed.

Next, the solid phase-bonded compound from which the protective group X has been removed, is condensed with the compound shown by the general formula (I). The conditions of this condensation reaction are identical to those of the above condensation reactions, except the solid phase resin or solid phase compound is replaced by the solid phase-bonded compound.

The compound shown by the general formula (i) is obtained by performing the removal of the protective group X of the above solid phase-bonded compound, and the condensation of the solid phase-bonded compound from which the protective group X has been removed with the compound shown by the general formula (I), n3-1 times.

### Step 1-2

In the step 1-2, the compound shown by the following general formula (ii) is synthesized using the compound shown by the general formula (i) obtained in the step 1-1, and the compound shown by the following general formula (II).

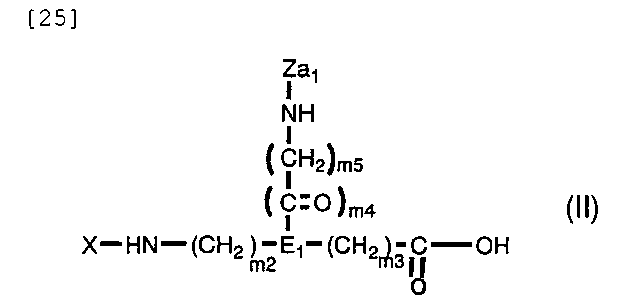

In the formula (II), m2-m5, E1 and X are identical to the above. Za1 is a protective group different from X. The compound shown by the general formula (II) is a publicly known compound, or a compound manufactured according to a publicly known manufacturing method.

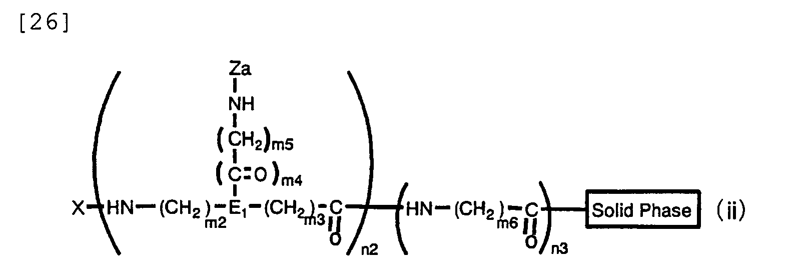

In the formula (ii), n2, n3, m2-m6, X, E1, Za1 and Solid Phase are identical to the above.

The protective group Za1 may be selected from among those protective groups shown above which are different from X. For example, if the protective group X is Boc or Fmoc, it is preferred that the protective group R is Aloc shown below.

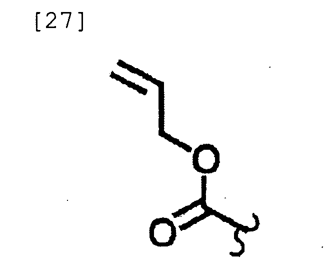

In the second step, first, the protective group X of the solid phase-bonded compound is removed.
The protective group X may be removed under identical conditions to those of the above step 1-1.

Next, a condensation reaction is performed between the solid phase-bonded compound from which the protective group X has been removed and the compound shown by the general formula (II).

The condensation between the solid phase-bonded compound from which the protective group X has been removed and the compound shown by the general formula (II) may be performed under identical conditions to those of the condensation reaction of the above step 1-1. Specifically, under the condensation reaction conditions of the above step 1-1, the condensation reaction of the step 1-2 is performed by substituting the solid resin or solid phase compound by the solid phase-bonded compound, and substituting the compound shown by the general formula (I), by the compound shown by the general formula (II).

By performing the removal of the protective group X of the solid phase-bonded compound, and the condensation of the solid phase-bonded compound from which the protective group X has been removed with the compound shown by the general formula (II) a total of n2 times, the compound shown by the general formula (ii) can be obtained.

### Step 1-3

In the step 1-3, the compound shown by the following general formula (iii) is synthesized using the compound shown by the general formula (ii) obtained in the step 1-2 and the compound shown by the general formula (III) below.

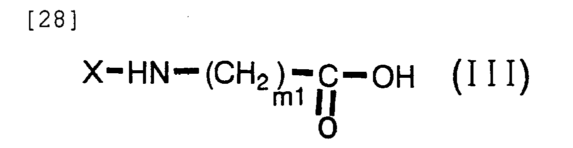

In the formula (III), m1 and X are identical to the above. The compound shown by the general formula (III) is a compound known in the art, or a compound manufactured according to a method known in the art.

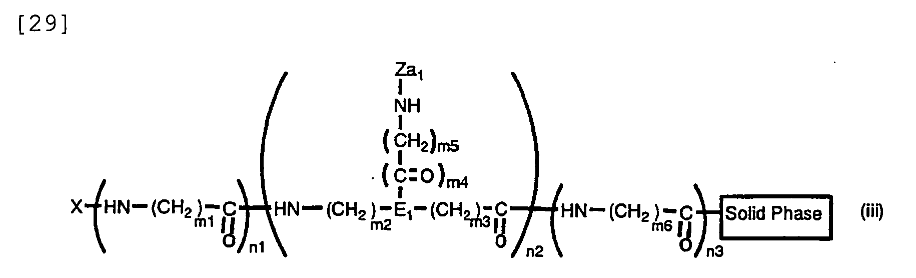

In the formula (iii), n1-n3, m1-m6, E1, X, Za1 and Solid Phase are identical to the above.

In the step 1-3, first, the protective group X of the solid phase-bonded compound is removed. The removal of the protective group X is performed under identical conditions to those of the first step.

Next, the solid phase-bonded compound from which the protective group X has been removed is made to undergo condensation with the compound shown by the following general formula (III).

The condensation of the solid phase-bonded compound from which the protective group X has been removed with the compound shown by the following general formula (III), is performed under identical conditions to those of the above step 1-1. Specifically, under the condensation reaction conditions of the above step 1-1, the condensation reaction of the step 1-3 is performed by substituting the solid resin or solid phase compound by the solid phase-bonded compound, and substituting the compound shown by the general formula (I), by the compound shown by the general formula (III).

By performing the removal of the protective group X of the solid phase-bonded compound, and the condensation of the solid phase-bonded compound from which the protective group X has been removed with the compound shown by the general formula (II) a total of n1 times, the compound shown by the general formula (iii) can be obtained.

### Step 1-4

In the step 1-4, the compound shown by the following formula (iv) is obtained by condensing one of the compounds shown by the following general formula (IV), or 1-10 of the compounds shown by the general formula (IV) and 1-100 of the compounds shown by the following general formula (V), with the compound shown by the general formula (iii) obtained in the step 1-3.

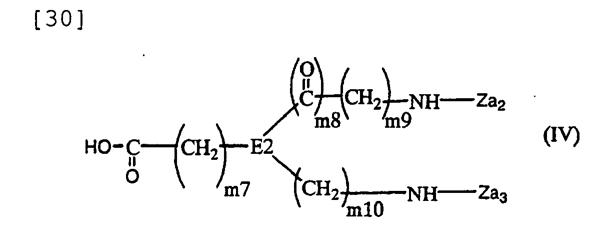

In the formula (IV), m7-m10 are identical to the above. Za2 and Za3 are protective groups which may be respectively identical or different, and are different from X, but may be identical to Za1.

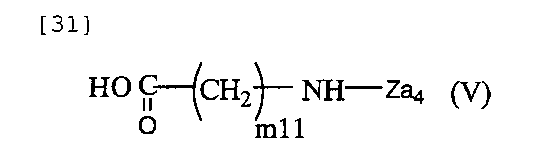

In the formula (V), m11 is identical to the above. Za4 is a protective group different from X, and may be identical to Za1, Za2, and Za3.

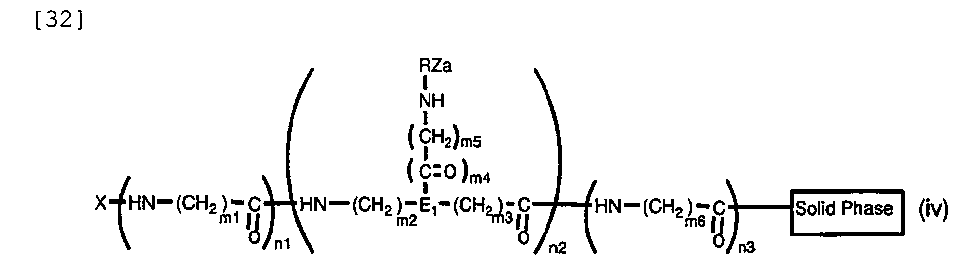

In the formula (iv), n1-n3, m1-m6, X, E1, and Solid Phase are identical to the above.

RZa denotes one of the following groups (a') or (b'):
(a') has one of the basic units D shown by the following general formula (D), wherein the protective groups Za2, Za3 are bonded to two nitrogen atoms at the termini of the basic unit D.
(b') is a group having 1-10 of the basic units D and 1-100 of the basic units G as a structural unit, having a branched structure wherein this structural unit is repeatedly bonded via a peptide linkage,
and wherein nitrogen atoms which do not form a peptide linkage of this group are protected by the protective groups Za3, Za4 or Za2, Za3, and Za4.

If a different protective group is used as Za2, Za3 and Za4 bonded to the basic unit D and basic unit G used in this step, only the protective group bonded to the amino group used in the condensation reaction may selectively be removed, and the protective groups bonded to other amino groups retained. Therefore, by suitably selecting identical or different protective groups as the protective groups Za2, Za3, Za4, in the compound having the general formula (I) which is the target compound, R may have any desired branched structure.

As one embodiment of the invention, the protective groups Za1, Za2, Za3 and Za4 may be identical.

In the step 1-4, first, the protective group Za1 bonded to the amino group used in the condensation reaction in the solid phase-bonded compound, is removed. The removal of the protective group Za1 may be performed under identical conditions to those of the above step 1-1.

Next, the solid phase-bonded compound from which the protective group Za1 has been removed as described above, is condensed with the compound shown by the above general formula (IV) or the compound shown by the general formula (V).

The condensation between the solid phase-bonded compound from which the protective group Za1 has been removed and the compound shown by the above general formula (IV) or the compound shown by the general formula (V), may be performed under identical conditions to those of the condensation reaction of the above step 1-1. Specifically, in the condensation reaction of the step 1-1, the solid phase resin or solid phase compound is replaced by the solid phase-bonded compound. Further, by substituting the compound shown by the general formula (I) by the compound shown by the above general formula (IV) or the compound shown by the general formula (V), the condensation of the step 1-4 can be performed.

Further, if required, the removal of the protective groups Za2, Za3 and/or Za4 of the solid phase-bonded compound, and the condensation of the solid phase-bonded compound from which the protective groups have been removed with the compound shown by the above general formula (IV) or the compound shown by the general formula (V), are performed a predetermined number of times by an identical method. In this way, the compound shown by the general formula (iv) can be obtained. For example, by performing the removal of the above protective group Za1 and the condensation of the compound shown by the above general formula (IV) once, a compound wherein R is the group shown by (a) can be synthesized. By performing 1) the removal of the above protective group Za1 and condensation of the compound shown by the above general formula (IV), 2) the removal of the above protective groups Za2, Za3 and condensation of the compound shown by the above general formula (V), 3) the removal of the above protective group Za4 and condensation of the compound shown by the above general formula (IV), and 4) the removal of the above protective groups Za2, Za3 and the condensation of the compound shown by the above general formula (V) in sequence, a compound wherein R is a group shown by (b1) can be synthesized.

### Step 1-5

Next, one, two or more of the protective groups Za2, Za3, Za4 and X are removed from the compound shown by the general formula (iv) obtained in the step 1-4, and a photosensitizing group is bonded to the nitrogen atoms to which the protective groups were bonded.

The method of removing the protective groups depends on the type of protective group to be removed in an identical way to the above.

The bonding of the photosensitizing group to the above nitrogen atoms may be performed by a reaction known in the art, such as condensation for example.

### Step 1-6

Next, the solid phase resin or solid phase compound is separated from the compound obtained in the above step 1-5, a hydroxyl group or an amino group is bonded to the carbon atoms to which the solid phase resin or solid phase compound was bonded, the protective groups not removed in the step 1-5 are removed, and a hydrogen atom is bonded to the nitrogen atoms to which the protective groups were bonded.

The removal of the protective groups and bonding of the hydrogen atom to the nitrogen atoms may be performed by methods known in the art.

If a Merrifield resin is used as the solid phase resin or solid phase compound, the compound having the general formula (1) wherein Y is a carboxyl group may be obtained by exposing it to highly acidic conditions. If on the other hand for example a MBHA resin is used as the solid phase resin or solid phase compound, the compound having the general formula (1) wherein Y is a hydroxyl group may be obtained by exposing it to highly acidic conditions.

Further, as a second method of manufacturing the compound shown by the general formula (1), the steps 2-1 to 2-6 shown below may be performed in sequence. Step 2-1 - Step 2-6 will now be described in detail for each step.

### Step 2-1

In the step 2-1, the compound shown by the general formula (i) is synthesized using the compound solid phase resin or solid phase compound shown by the above general formula (I).

First, the compound shown by the above general formula (I) is condensed with the solid phase resin or solid phase compound to obtain the solid phase-bonded compound, and the protective group X of this solid phase-bonded compound is then removed. The removal of the protective group X may be performed under identical conditions to those of the step 1-1.

Next, the condensation reaction of the phase-bonded compound from which the protective group X has been removed with the compound shown by the general formula (I), is performed n3-1 times. This condensation reaction is performed under identical conditions to those of the step 1-1. In this way, the compound shown by the general formula (i) can be obtained.

### Step 2-2

In the step 2-2, the compound shown by the following general formula (II') is synthesized by condensing one of the compounds shown by the general formula (IV), or 1-10 of the compounds shown by the general formula (IV) and 1-100 of the compounds shown by the general formula (V), with the compound shown by the above general formula (II).

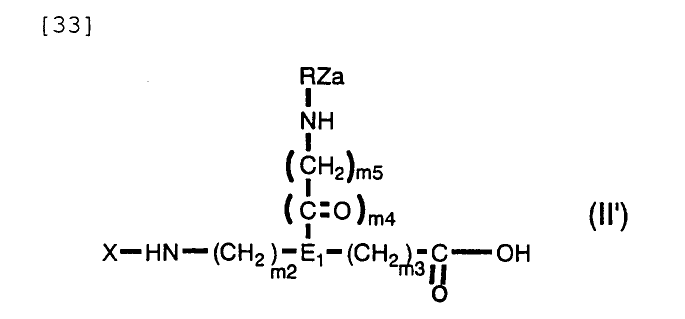

In the formula (II'), m2-m5, X, E1, and RZa are identical to the above.

In the step 2-2, the carboxyl group of the compound shown by the general formula (II) is preferably protected by a protective group different from X, Za1, Za2, Za3, and Za4.

First, the protective group Za1 of the compound shown by the general formula (II) is removed. The removal of the protective group Za1 may be performed under identical conditions to those of the step 1-4.

Next, the compound shown by the general formula (II) from which the protective group Za has been removed, is made to undergo a condensation reaction with the compound shown by the general formula (IV) or the compound shown by the general formula (V). This condensation reaction may be performed under identical conditions to those of the step 1-4.

Further, if required, the compound shown by the general formula (II') may be obtained by repeating the removal of the protective groups Za2, Za3, and/or Za4 of the solid phase-bonded compound, and the condensation of the solid phase-bonded compound from which the protective groups have been removed with the compound shown by the general formula (IV) or the compound shown by the general formula (V), a predetermined number of times by an identical method to the above.

### Step 2-3

In the step 2-3, the compound shown by the following general formula (ii') is synthesized using the compound shown by the general formula (i) obtained in the step 2-1, and the compound shown by the general formula (II') obtained in the step 2-2.

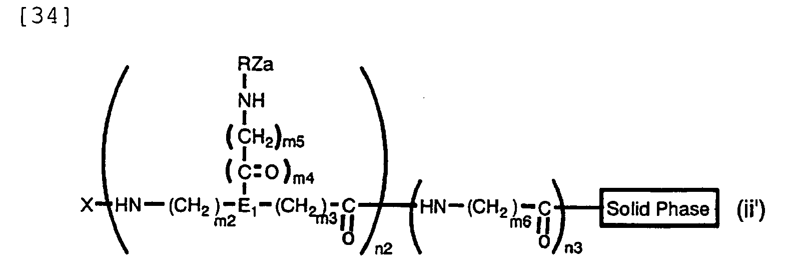

In the formula (ii'), n2, n3, m2-m6, RZa, E1, and Solid Phase are identical to the above.

First, the protective group X of the solid phase-bonded compound is removed.
The protective group X is removed under identical conditions as those of the step 1-2.

Next, the solid phase-bonded compound from which the protective group X has been removed is made to undergo a condensation reaction with the compound shown by the general formula (III).
This condensation reaction is performed under identical conditions to those of the step 1-2.

By repeating the removal of the protective group X from the solid phase-bonded compound, and the condensation reaction of the solid phase-bonded compound from which the protective group X has been removed with the compound shown by the general formula (II'), a total of n2 times, the compound shown by the following general formula (ii') is obtained.

### Step 2-4

In the step 2-4, the compound shown by the general formula (iv) is synthesized using the compound shown by the general formula (ii') obtained in the step 2-3 with the compound shown by the general formula (III).

First, the protective group X of the solid phase-bonded compound is removed. The protective group X is removed under identical conditions as those of the step 1-3.

Next, the solid phase-bonded compound from which the protective group X has been removed is made to undergo a condensation reaction with the compound shown by the general formula (III).
This condensation reaction is performed under identical conditions to those of the step 1-3.

By performing the removal of the protective group X of the solid phase-bonded compound, and the condensation of the solid phase-bonded compound from which the protective group X has been removed with the compound shown by the general formula (III), a total of n1 times, the compound shown by the general formula (iv) is obtained.

### Step 2-5

Next, one, two or more of the protective groups Za2, Za3, Za4 and X are removed from the compound shown by the general formula (iv) obtained in the step 2-4, and a photosensitizing group is bonded to the nitrogen atoms to which the protective groups were bonded. The step 2-5 is performed under identical conditions to those of the step 1-5.

### Step 2-6

Next, the solid phase resin or solid phase compound is separated from the compound obtained in the above step 2-5, a hydroxyl group or an amino group is bonded to the carbon atoms to which the solid phase resin solid phase compound was bonded, the protective groups not removed in the step 2-5 are removed, and a hydrogen atom is bonded to the nitrogen atoms to which the protective groups were bonded. The step 2-6 is performed under identical conditions to those of the step 2-5.

### 2. Photoelectrode

When the compound shown by the above-mentioned general formula (1) is used as a sensitizing dye of a dye-sensitized solar cell, it shows excellent durability due to the fact that it is not easily desorbed from the semiconductor electrode. Furthermore, it also shows excellent photovoltaic conversion efficiency.

Therefore, the present invention further provides an photoelectrode using the compound shown by the above-mentioned general formula (1).

Specifically, the photoelectrode of the present invention includes a conductive substrate, and a porous semiconductor layer formed on the conductive substrate on which the compound shown by the general formula (I) is adsorbed.

In general, the conductive substrate forming the photoelectrode of the invention comprises a supporting substrate and a conductive layer formed thereupon. When light is incident from the conductive layer substrate side, the supporting substrate and conductive layer normally transmit the light. The supporting substrate may for example be a glass substrate, a plastic substrate, etc.
The thickness of the supporting substrate is not particularly limited, but maybe suitably set within a range which gives a desired strength to the photoelectrode. The conductive layer may be a film of conductive material such as ITO, SnO₂, ZnO or the like. This conductive layer may be formed by a method known in the art such as spray coating, vacuum deposition or sputtering, and its thickness is preferably about 0.1 µm - 5 µm.

If light is not made to be incident from the conductive substrate side, the conductive substrate which constitutes the photoelectrode of the invention may be a metal substrate such as platinum, silver, copper, nickel, titanium, tungsten, molybdenum, indium, aluminum and the like.

The photoelectrode of the invention is formed by the porous semiconductor layer on which the compound shown by the general formula (1) is adsorbed, on the conductive layer of the conductive substrate.

The semiconductor used for the porous semiconductor layer is not particularly limited, specific examples being, titanium oxide, barium titanate, strontium titanate, tungstic oxide, zinc oxide, tin oxide, plumbous sulfide, niobium oxide, zirconium oxide, cerium oxide, oxidation silicon, aluminum oxide, iron oxide, oxidation nickel, cadmium sulfide, zinc sulfide, and indium phosphide.
One of these may be used alone, or two or more may be used together. Among these, titanium oxide is preferred, anatase type TiO₂ being particularly preferred.

The semiconductor used for the porous semiconductor layer may for example have a particle diameter of 5-500 nm.

The thickness of the porous semiconductor layer constituting the semiconductor electrode may for example be set to about 0.1-100 µm.

The forming of the porous semiconductor layer on the conductive substrate may be performed by a method known in the art such as the doctor blade method, spin coat method, and screen printing.

The adsorption of the compound shown by the general formula (1) on the porous semiconductor layer may be performed by for example immersing the conductive substrate on which the porous semiconductor layer is formed, in a solution containing the compound shown by the general formula (1).

The solution containing the compound shown by the general formula (1) is prepared using an organic solvent, such as methanol, ethanol, toluene, acetonitrile, and chloroform.
Moreover, in this solution, the concentration of the compound shown by the general formula (1) is usually adjusted to 1x10¹-1x10¹⁰mol/L.

When the conductive substrate on which the porous semiconductor layer is formed is immersed in the solution containing the compound shown by the general formula (1), the temperature and immersion time are set to appropriate values. The immersion may be performed once or several times, and drying may be performed after the immersion step if required.

### 3. Dye-sensitized Solar Cell

The present invention further provides a dye-sensitized solar cell using the above-mentioned photoelectrode. The photoelectrode of the invention achieves an excellent photovoltaic conversion efficiency due to the compound shown by the general formula (1), this compound being strongly bonded to the porous semiconductor layer. Therefore, the dye-sensitized solar cell using the above photoelectrode exhibits excellent photovoltaic conversion efficiency and durability.

Specifically, the dye-sensitized solar cell of the present invention includes the above photoelectrode, and an electrode opposite to this photoelectrode with an electrolyte layer sandwiched therebetween.

If light is made to be incident from the photoelectrode side, the counter-electrode need not be transparent. If light is made to be incident from the counter-electrode side, the counter-electrode normally needs to be transparent.

The counter-electrode is not particularly limited provided that it can supply electrons to the electrolyte layer. Specifically, the counter-electrode may be formed from a supporting substrate, and a conductive layer and catalyst layer alternately laminated on this supporting substrate.
The supporting substrate may be a glass substrate, a plastic substrate or the like.
The thickness of the supporting substrate is not particularly limited, and is suitably set within a range which gives a desired strength to the photoelectrode.

The conductive layer forming the counter-electrode may be formed of a metal for example such as gold, platinum, silver, copper, aluminium, titanium, tantalum and tungsten, an elemental semiconductor such as silicon or germanium, and a conductive material such as ITO, SnO₂ or ZnO.
These conductive layers are formed by a method known in the art, and the thickness of the conductive layer of the counter-electrode is preferably about 0.1 µm-5 µm.

The catalyst layer may be a material such as platinum, carbon nanotube, fullerene and the like. Among these, platinum is preferably used as the catalyst layer, and if platinum is used as the catalyst layer, the thickness of the catalyst layer may be set to for example about 1 nm-100 nm.

If the electrical conductivity of the catalyst layer is sufficiently high, the conductive layer of the counter-electrode need not be provided.

The electrolyte layer is not particularly limited provided that it can transfer electrons between the photoelectrode and the counter-electrode, but a preferable example is an electrode formed from a liquid electrolyte containing a redox electrolyte. The redox electrolyte is not particularly limited provided that it is one that can be used in batteries and solar cells. Specifically, it may contain redox pairs, such as I⁻/I₃⁻, Br₃⁻/Br⁻, and quinone/hydroquinone. Among these, I⁻/I₃⁻ is preferred.

The solvent in which the redox electrolyte is dissolved is preferably electrically inert, has a high dielectric constant, and a low viscosity. Specific examples of this solvent are a nitrile solvent such as methoxypropionitrile acetonitrile, etc., a lactone solvent such as γ-butyrolactone valerolactone, etc., and a carbonate solvent such as ethylene carbonate, propylene carbonate, etc.

The electrolyte concentration in the liquid electrolyte depends on the type of electrolyte and solvent used, but may for example be 0.05-2 mol/L.

### 4. Dye-sensitized Solar Cell Module

The present invention further provides a dye-sensitized solar cell module using the above-mentioned dye-sensitized solar cell. Specifically, this dye-sensitized solar cell module uses the above dye-sensitized solar cell as a unit cell. This unit cell is formed from two or more, preferably 2-1000, and preferably 2-100 cells connected in series.

### Embodiments

Hereafter, the invention will be described in more detail referring to embodiments and experiments. However, it is to be understood that the invention is not to be construed as being limited in any way by these embodiments and experiments.

### First embodiment

The compound shown by the general formula 16 or 19 was obtained as follows.

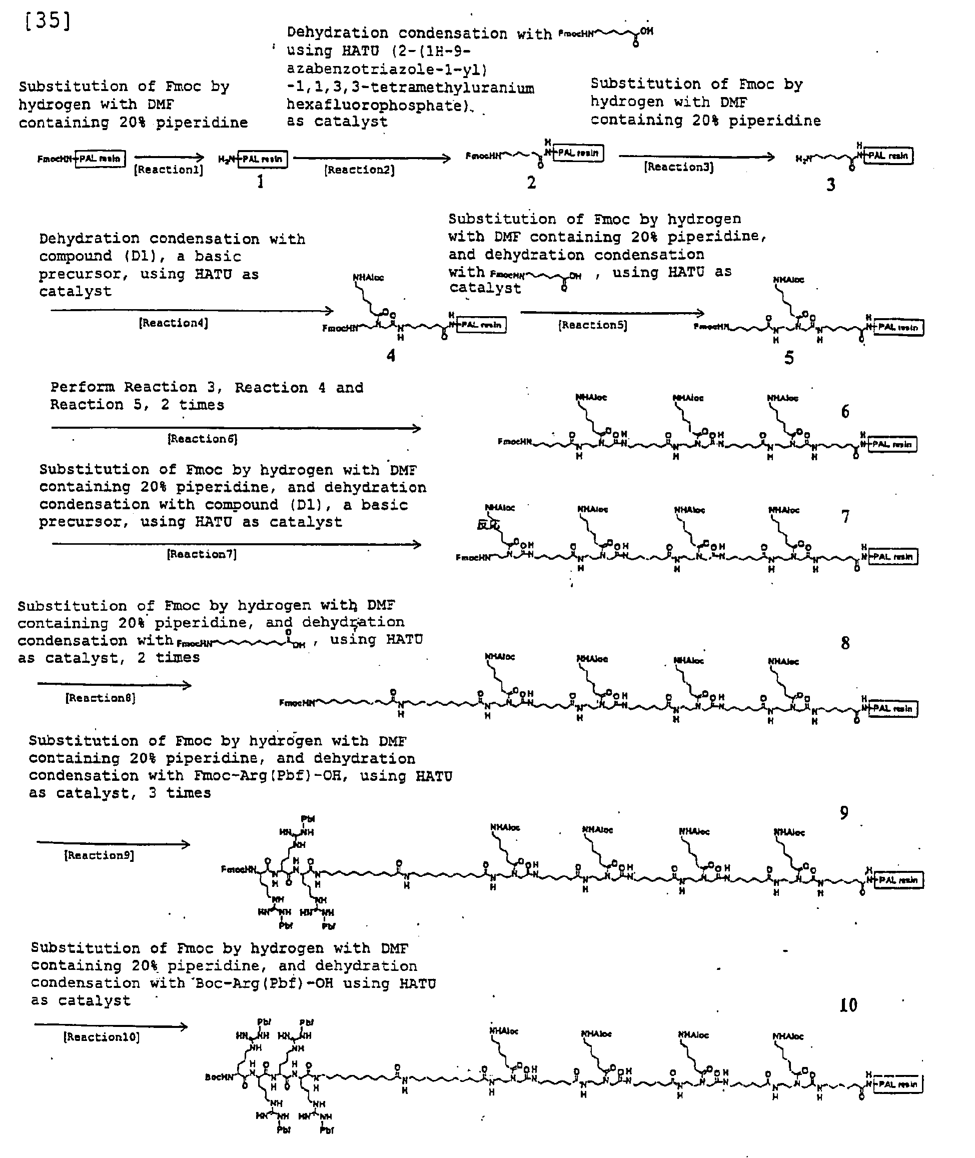

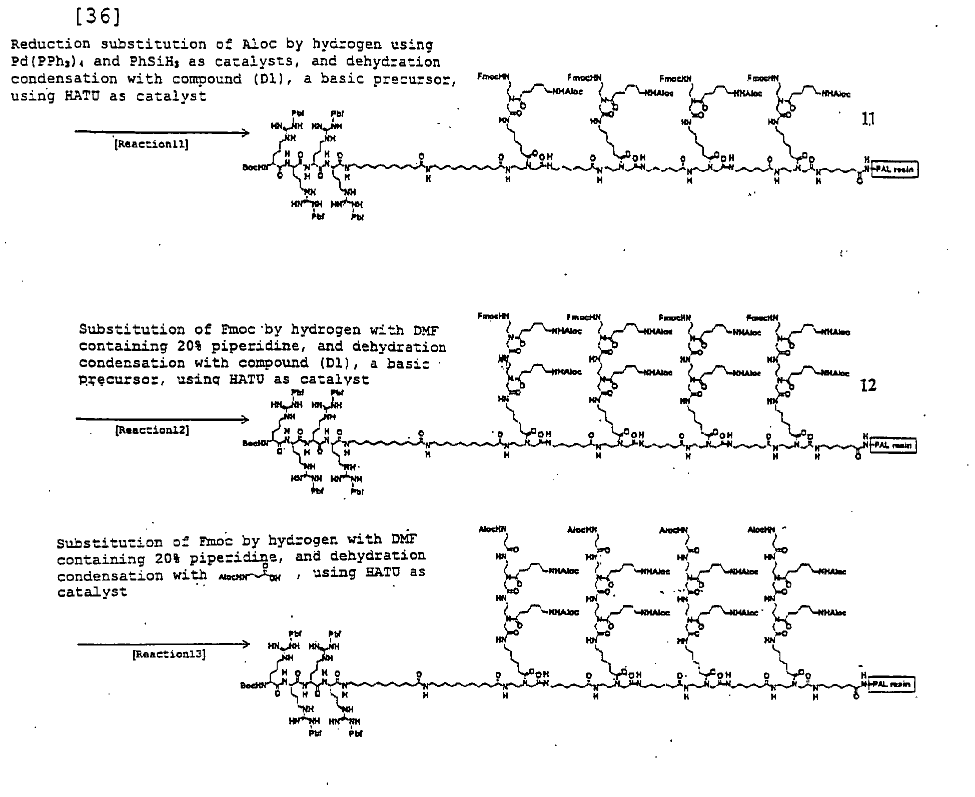

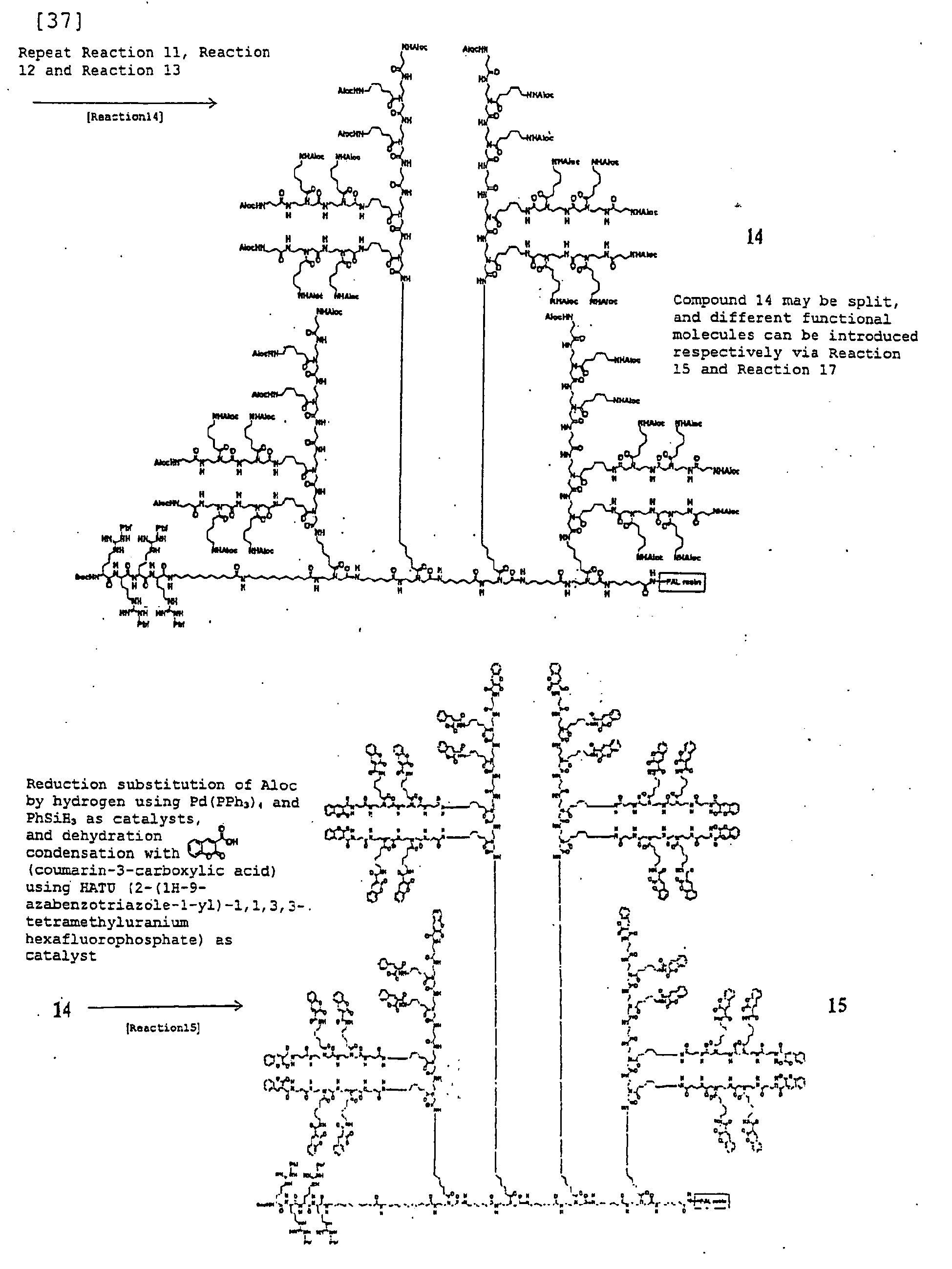

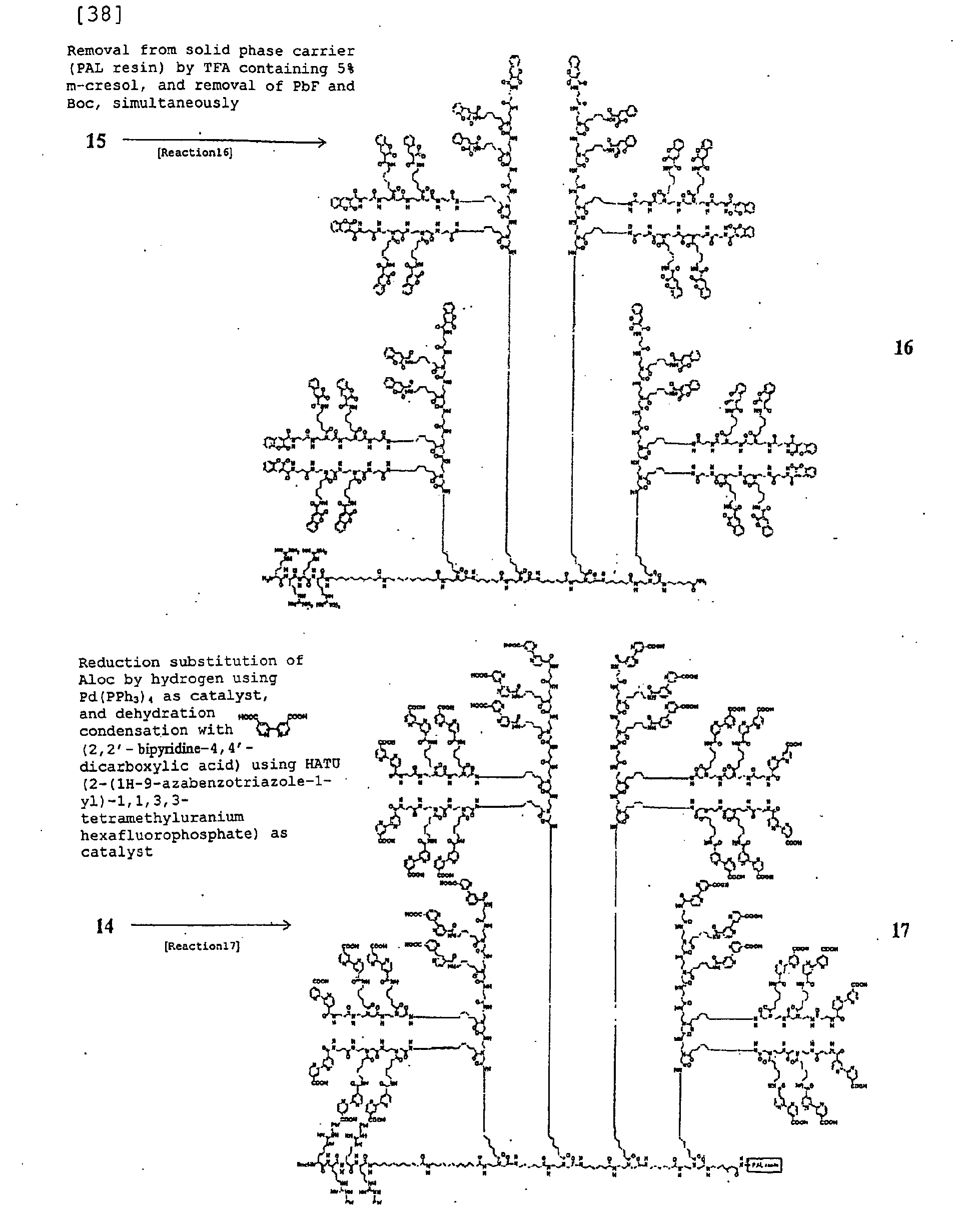

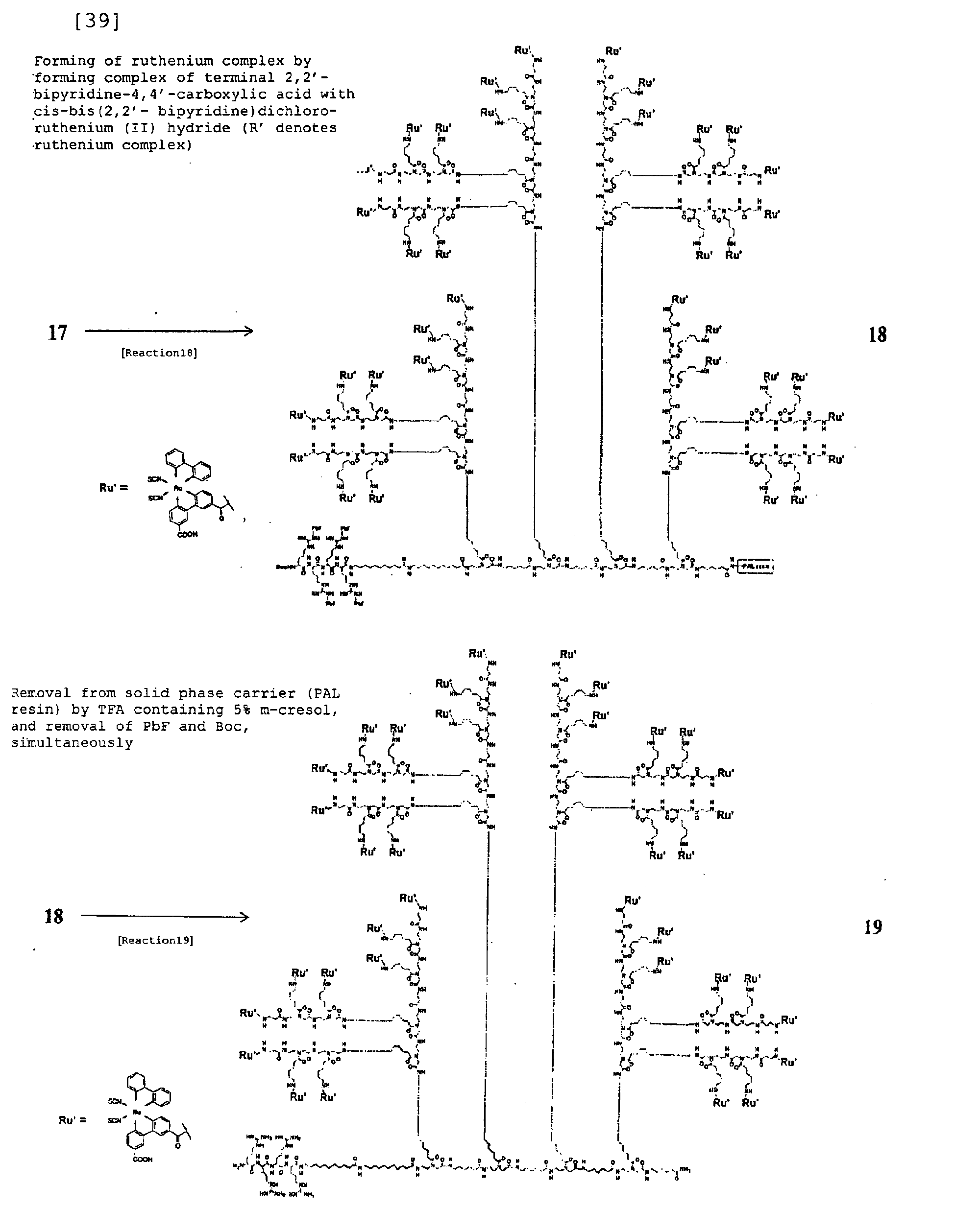

The specific details of the reactions 1-18 are as follows.

### [Reaction 1] Synthesis of compound shown by the formula 1: PAL

(5-(4'-aminomethyl-3',5'-dimethoxyphenoxy)valeric acid) PEG (polyethyleneglycol)-bonded resin protected by a Fmoc (9-fluorenyl carbomethoxy) group (350 mg) was stirred for 5 minutes in 5ml of 20% piperidine DMF solution. The above solution was removed by filtration, and by washing the remaining solid resin with DMF (N,N-dimethylformamide) and methylene chloride, the compound shown by the formula 1 was obtained (the removal of the Fmoc group was confirmed by the ninhydrin test).

### [Reaction 2] Synthesis of compound shown by the formula 2:

The compound shown by the formula 1 was stirred at room temperature for 2 hours in 2 ml of a NMP (N-methylpyrrolidone) solution in which 20 mg of Fmoc-C₅-OH (56.6 µmol), 26.0 mg of HATU (2-(1H-9-azabenzotriazole-1-yl)-1,1,3,3-tetramethyluranium hexafluorophosphate) and 16 µl of DIEA (diisopropylethylamine) had been dissolved. Next, the NMP solution was removed by filtration, the remaining solid phase resin was washed with DMF and methylene chloride, and the compound shown by the formula 2 was obtained (the dehydration condensation of free amino groups and Fmoc-C₅-OH was verified by the ninhydrin test).

### [Reaction 3] Synthesis of the compound shown by the formula 3:

The compound shown by the formula 2 (28.3 µmol) was stirred for 5 minutes at room temperature in 5 ml of 20% piperidine DMF solution. Next the reaction solution was removed by filtration, and the remaining solid phase resin was washed with DMF and methylene chloride,
and the compound shown by the formula 3 was obtained (the removal of the Fmoc group was confirmed by the ninhydrin test).

### [Reaction formula 4] Synthesis of the compound shown by the formula 4:

The compound shown by the formula 3 was stirred at room temperature for 30 minutes in 3 ml of a NMP solution in which 45.6 mg of the compound (D1) shown by the general formula (D1), 32.3 mg of HATU and 30 µl of DIEA had been dissolved.

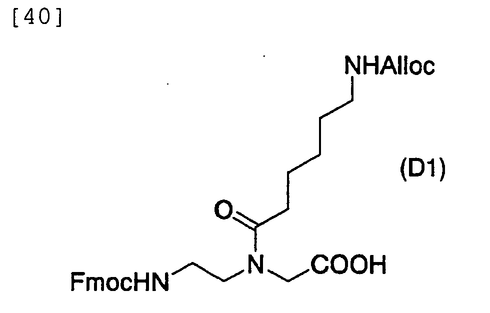

Next, the NMP solution was removed by filtration, the remaining solid phase resin was washed with DMF and methylene chloride, and the compound shown by the formula 4 was obtained (the dehydration condensation of free amino groups with the comound (D1) was confirmed by the ninhydrin test).

### [Reaction 5] Synthesis of the compound shown by the formula 5:

The compound shown by the formula 4 was stirred at room temperature for 5 minutes in 5 ml of 20% piperidine DMF solution. Next, the reaction solution was removed by filtration, and the remaining solid phase resin was washed with DMF and methylene chloride (the removal of the Fmoc group was confirmed by the ninhydrin test). Next, the compound obtained was stirred at room temperature for 30 minutes in 3 ml of a NMP solution in which 30 mg of Fmoc-C₅-OH, 32.3 mg of HATU and 30 µl of DIEA had been dissolved. Next, the NMP solution was removed by filtration, and the remaining solid phase resin was washed with DMF and methylene chloride, and the compound shown by the formula 5 was obtained (the dehydration condensation of free amino groups with Fmoc-C₅-OH was confirmed by the ninhydrin test).

### [Reaction 6] Synthesis of the compound shown by the formula 6:

The reaction 3, reaction 4 and reaction 5 were performed twice in the same way on the compound shown by the formula 5, and the compound shown by the formula 6 was obtained (the progress of each reaction was confirmed by the ninhydrin test).

### [Reaction 7] Synthesis of the compound shown by the formula 7:

The compound shown by the formula 6 was stirred at room temperature for 5 minutes in 5 ml of 20% piperidine DMF solution. Next, the reaction solution was removed by filtration, and the remaining solid phase resin was washed with DMF and methylene chloride (the removal of the Fmoc group was confirmed by the ninhydrin test). The compound obtained was stirred at room temperature for 30 minutes in 3 ml of a NMP solution in which 45.6 mg of the compound (D1), 32.3 mg of HATU and 30 µl of DIEA had been dissolved. Next, the NMP solution was removed by filtration, the remaining solid phase resin was washed with DMF and methylene chloride, and the compound shown by the formula 7 was obtained (the dehydration condensation of free amino groups with the comound (D1) was confirmed by the ninhydrin test).

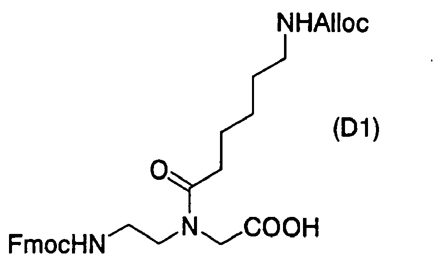

### [Reaction 8] Synthesis of the compound shown by the formula 8:

The compound shown by the formula 7 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). Next, the compound obtained was stirred at room temperature for 30 minutes in 3 ml of a NMP solution in which 36 mg of Fmoc-C₁₀-OH, 32.3 mg of HATU and 30 µl of DIEA had been dissolved. Next, the NMP solution was removed by filtration, and the remaining solid phase resin was washed with DMF and methylene chloride (the dehydration condensation of free amino groups with Fmoc-C₁₀-OH was confirmed by the ninhydrin test). The same procedure was performed in the same way as above on the compound thus obtained, and 356.2 mg of the compound shown by the formula 8 was obtained (the progress of each reaction was confirmed by the ninhydrin test).

### [Reaction 9] Synthesis of the compound shown by the formula 9:

The compound shown by the formula 8 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 27.5 mg of Fmoc-Arg(Pbf)-OH, 16.1 mg of HATU and 15 µl of DIEA had been dissolved. Next, the NMP solution was removed by filtration, and the remaining solid phase resin was washed with DMF and methylene chloride (the dehydration condensation of free amino groups with Fmoc-Arg(Pbf)-OH was confirmed by the ninhydrin test). This procedure was performed twice on the compound thus obtained, and the compound shown by the formula 9 was obtained (the progress of each reaction was confirmed by the ninhydrin test).

### [Reaction 10] Synthesis of the compound shown by the formula 10:

The compound shown by the formula 9 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 24.5 mg of Boc-Arg(Pbf)-OH, 16.1 mg of HATU and 15 µl of DIEA had been dissolved.
Next, the NMP solution was removed by filtration, the remaining solid phase resin was washed with DMF and methylene chloride, and the compound shown by the formula 10 was obtained (the dehydration condensation of free amino groups with Boc-Arg(Pbf)-OH was confirmed by the ninhydrin test).

### [Reaction 11] Synthesis of the compound shown by the formula 11:

The compound shown by the formula 10 was treated for 3 minutes with 1ml of a methylene chloride solution of PhSiH₃ (phenylsilane) (174.6 µl), Pd(PPh₃)₄ (19.6 mg) was added, and the mixture stirred for 30 minutes. The reaction solution was removed by filtration, the remaining solid phase resin was washed with methylene chloride, a 10% dioxane aqueous solution, DMF, and a 1% N,N'-diethyldithiocarbamic acid DMF solution, and 10' having a free amino group was obtained (the removal of the Fmoc group was confirmed by the ninhydrin test). 10' was stirred at room temperature for 30 minutes in 2 ml of a NMP solution in which 91.2 mg of the compound (D1), 64.6 mg of HATU and 60 µl of DIEA had been dissolved. Next, the NMP solution was removed by filtration, the remaining solid phase resin was washed with DMF and methylene chloride, and the compound shown by the formula 11 was obtained (the dehydration condensation of free amino groups with the compound (D1) was confirmed by the ninhydrin test).

### [Reaction 12] Synthesis of the compound shown by the formula 12:

The compound shown by the formula 11 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 91.2 mg of the compound (D1), 64.6 mg of HATU and 60 µl of DIEA had been dissolved. Next, the NMP solution was removed by filtration, the remaining solid phase resin was washed with DMF and methylene chloride, and the compound shown by the formula 12 was obtained (the dehydration condensation of free amino groups with the compound (D1) was confirmed by the ninhydrin test).

### [Reaction 13] Synthesis of the compound shown by the formula 13:

The compound shown by the formula 12 was stirred at room temperature for 5 minutes in 5 m1 of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 29.4 mg of Aloc-C₂-OH, 64.6 mg of HATU and 60 µl of DIEA had been dissolved. Next, the NMP solution was removed by filtration, the remaining solid phase resin was washed with DMF and methylene chloride, and the compound shown by the formula 13 was obtained (the dehydration condensation of free amino groups with Aloc-C₂-OH was confirmed by the ninhydrin test).

### [Reaction 14] Synthesis of the compound shown by the formula 14 :

The compound shown by the formula 13 was treated for 3 minutes with 1ml of a methylene chloride solution of PhSiH₃ (phenylsilane) (523.8 µl), Pd(PPh₃)₄ (tetrakis(triphenylphospine) palladium (O)) (58.8 mg) was added, and the mixture stirred for 30 minutes. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Fmoc group was confirmed by the ninhydrin test).
The compound obtained was stirred at room temperature for 30 minutes in 2 ml of a NMP solution in which 273.7 mg of the compound (D1), 193.7 mg of HATU and 180 µl of DIEA had been dissolved. Next, the NMP solution was removed by filtration, and the remaining solid phase resin was washed with DMF and methylene chloride (the dehydration condensation of free amino groups with the compound (D1) was confirmed by the ninhydrin test). Next, the compound obtained was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound thus obtained was stirred at room temperature for 30 minutes in 2 ml of a NMP solution in which 273.7 mg of the compound (D1), 193.7 mg of HATU and 180 µl of DIEA had been dissolved. Next, the NMP solution was removed by filtration, and the remaining solid phase resin was washed with DMF and methylene chloride (the dehydration condensation of free amino groups with the compound (D1) was confirmed by the ninhydrin test). The compound obtained was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 88.2 mg of Aloc-C₂-OH, 193.7 mg of HATU and 180 µl of DIEA had been dissolved. Next, the NMP solution was removed by filtration, the remaining solid phase resin was washed with DMF and methylene chloride, and the compound shown by the formula 14 (218 mg) was obtained (the dehydration condensation of free amino groups with Aloc-C₂-OH was confirmed by the ninhydrin test).

### [Reaction 15] Synthesis of the compound shown by the formula 15:

30 mg of the compound shown by the formula 14 was stirred for 3 minutes in 1ml of a methylene chloride solution of PhSiH₃ (216 µl), Pd(PPh₃)₄ was added, and the mixture stirred for 30 minutes. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Fmoc group was confirmed by the ninhydrin test). The compound obtained was stirred at room temperature for 16 hours in NMP solution (1 ml) containing coumarin-3-carboxylic acid (20.5 mg), HATU (41 mg) and DIEA (100 µl). Next, the NMP solution was removed by filtration, the remaining solid phase resin was washed with DMF and methylene chloride, and the compound shown by the formula 15 was obtained (the dehydration condensation of free amino groups with coumarin-3-carboxylic acid was confirmed by the ninhydrin test).

### [Reaction 16] Synthesis of the compound shown by the formula 16:

The compound shown by the formula 15 was stirred away from light for 2 hours in 0.5ml of TFA solution (TFA solution of 5% m-cresol). Next, the TFA solution was recovered by filtration, and the remaining solid phase resin was washed by 0.1 ml of TFA. The filtrate was dripped into cold diethyl ether (10ml), and the precipitate was collected by centrifugal separation. The residue was dissolved in a small amount of methanol, diethyl ether was added to separate it out, and recovery by centrifugal separation was performed twice. Residual ether was removed, and after drying, 1.3 mg of the compound shown by the formula 16 was obtained.

### [Reaction 17] Synthesis of the compound shown by the formula 17:

30 mg of the compound shown by the formula 14 was stirred for 3 minutes in 1 ml of a methylene chloride solution of PhSiH₃ (216 µl), Pd(PPh₃)₄ (16.3 mg) was added, and the mixture stirred for 30 minutes. Next, the reaction solution was removed by filtration, the remaining solid phase resin was washed with methylene chloride, 10% dioxane aqueous solution, DMF, and 1% N,N'-diethyldithiocarbamic acid DMF solution, and 14' having a free amino group was obtained (the removal of the Fmoc group was confirmed by the ninhydrin test). 14' was stirred at room temperature for 16 hours in 1 ml of a NMP solution containing 2,2'-bipyridine-4,4'-dicarboxylic acid (bpy) (79 mg), HATU (41 mg) and DIEA (170 µl). Next, the NMP solution was removed by filtration, the remaining solid phase resin was washed with DMF and methylene chloride, and the compound shown by the formula 17 was obtained (the dehydration condensation of free amino groups with 2,2'-bipyridine-4,4'-dicarboxylic acid was confirmed by the ninhydrin test).

### [Reaction 18] Synthesis of the compound shown by the formula 18:

30 mg (2 µmol (72 µmol: free amino groups), 1.0 eq) of the compound shown by the formula 17 was introduced into an eggplant flask, mixed with 30 ml of an ethanol aqueous solution (ethanol:distilled water =1:7), cis-bis (2,2'-bipyridine) dichloro-ruthenium (II) hydride (181.5 mg, 375 µmol, 5.0 eq) was added, the mixture heated on an oil bath (about 80°) using a reflux condenser, and stirred for 16 hours. The reaction solution was removed by filtration, the residual solid phase resin was washed with 50% methanol aqueous solution, DMF and methylene chloride, and the compound shown by the formula 18 was obtained.

### [Reaction 19] Synthesis of the compound shown by the formula 19:

The compound shown by the formula 18 was treated at room temperature for 1.5 hours away from light in 0.3 ml of a TFA solution (TFA solution of 5% m-cresol), the TFA solution was recovered by filtration, and the residual solid phase resin was washed with 0.2 ml TFA. The filtrate was dripped into cold diethyl ether (10 ml), and the precipitate was recovered by centrifugation. The residue was dissolved in a small amount of methanol, diethyl ether was added to separate it out, and recovery by centrifugal separation was performed 3 times. Residual ether was removed, and after drying, 0.6 mg of the compound shown by the formula 19 was obtained.

### Second embodiment

Similarly, the compound shown by a formula 30 was obtained in accordance with the following methods.

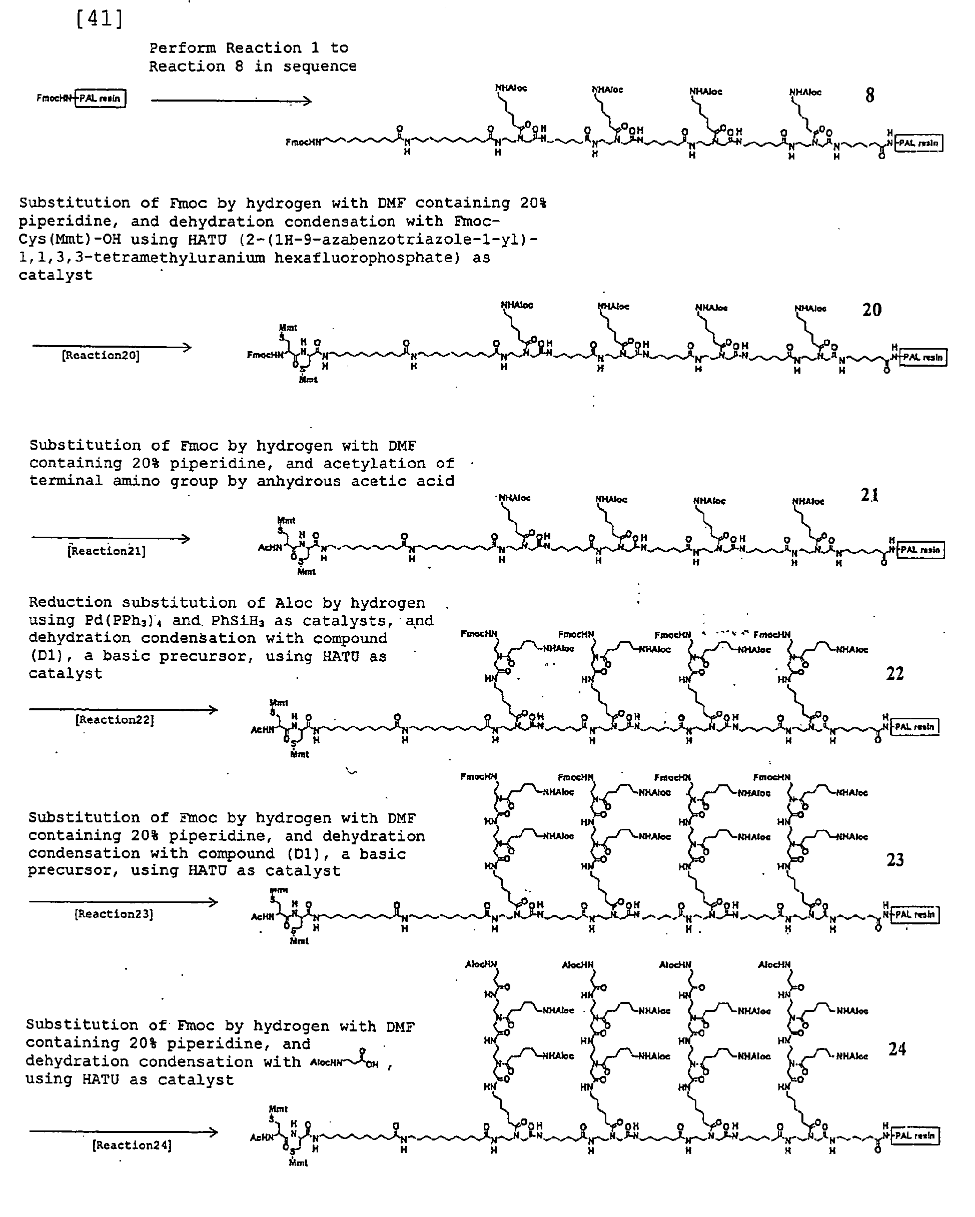

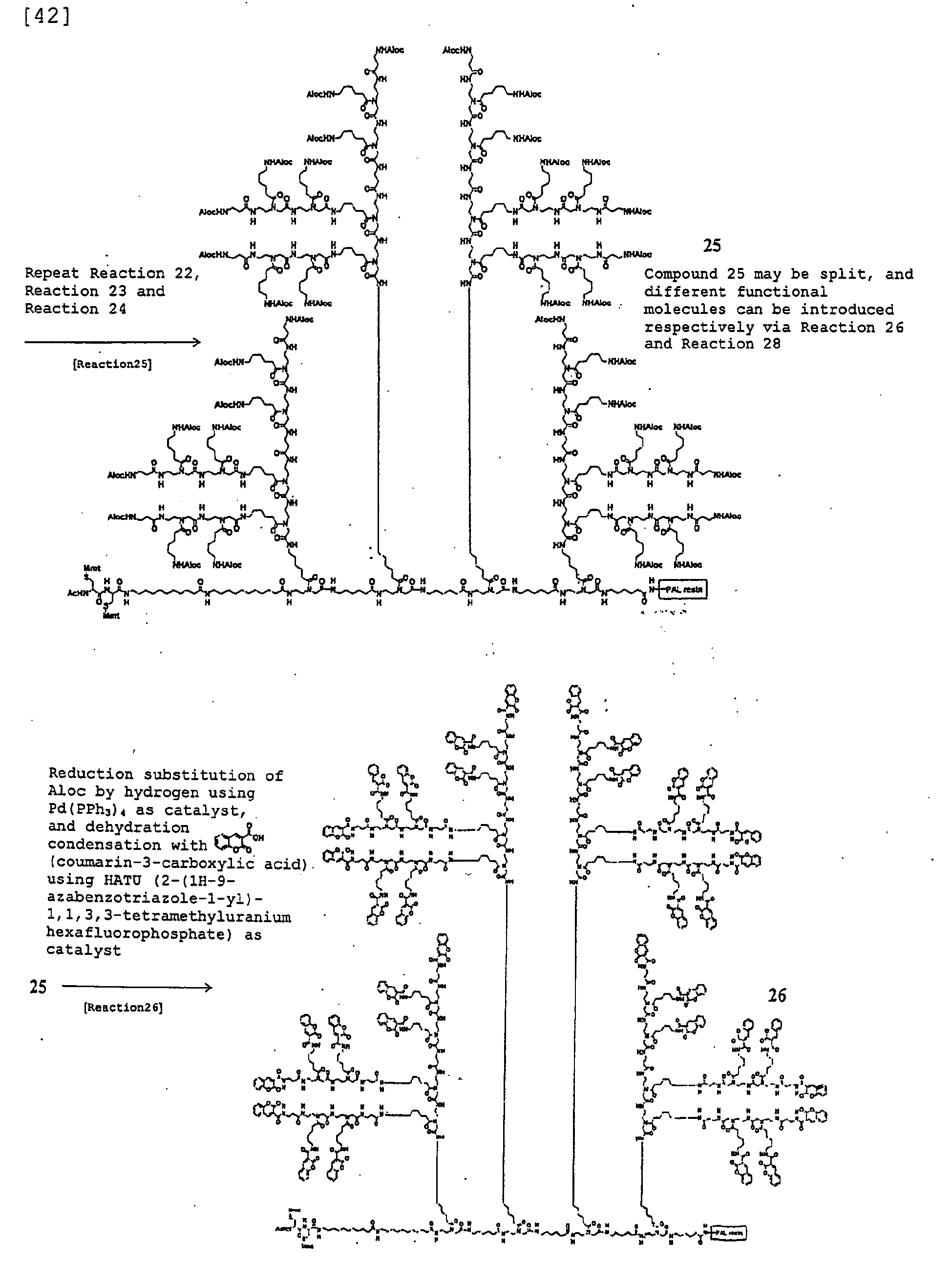

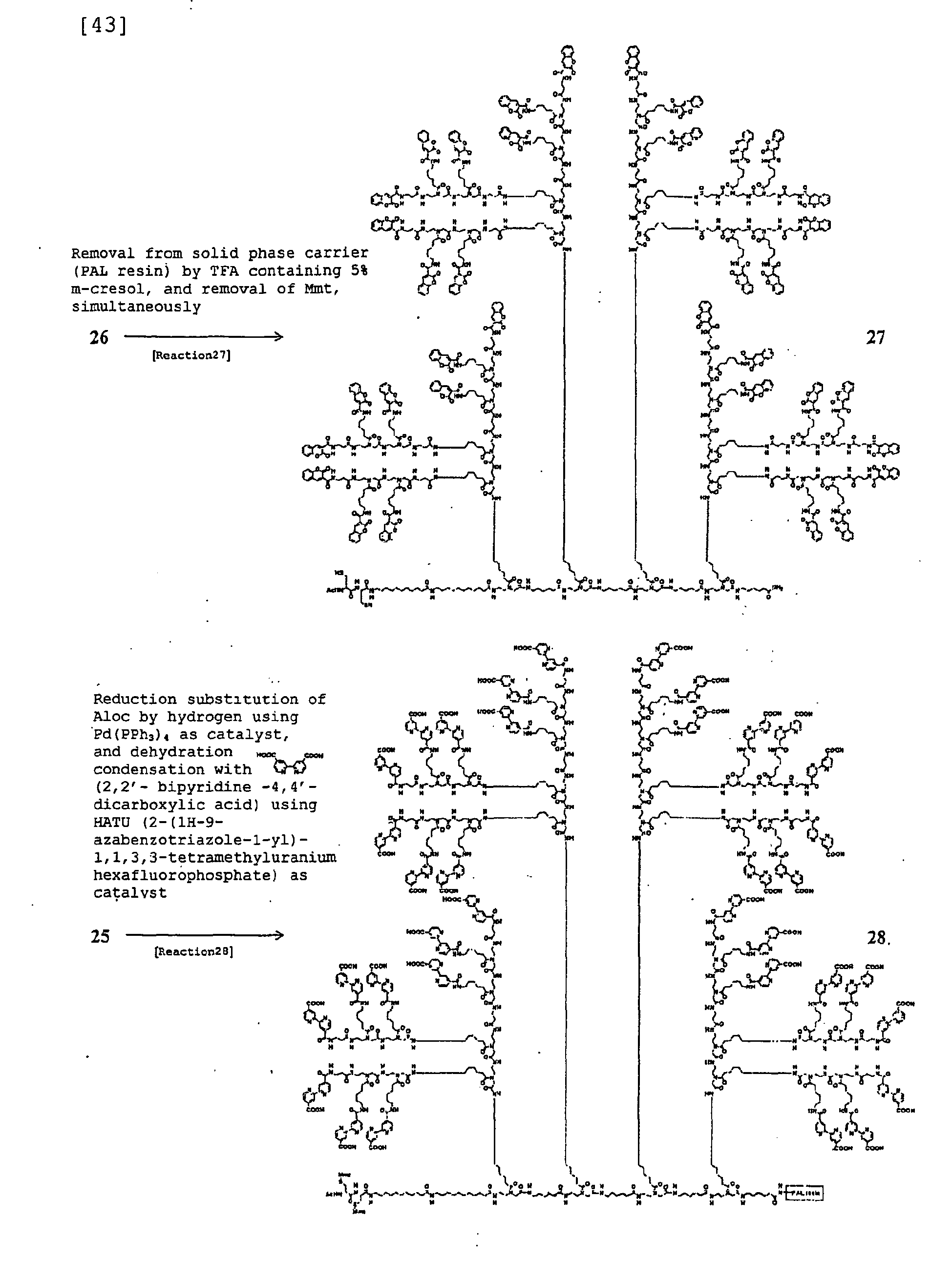

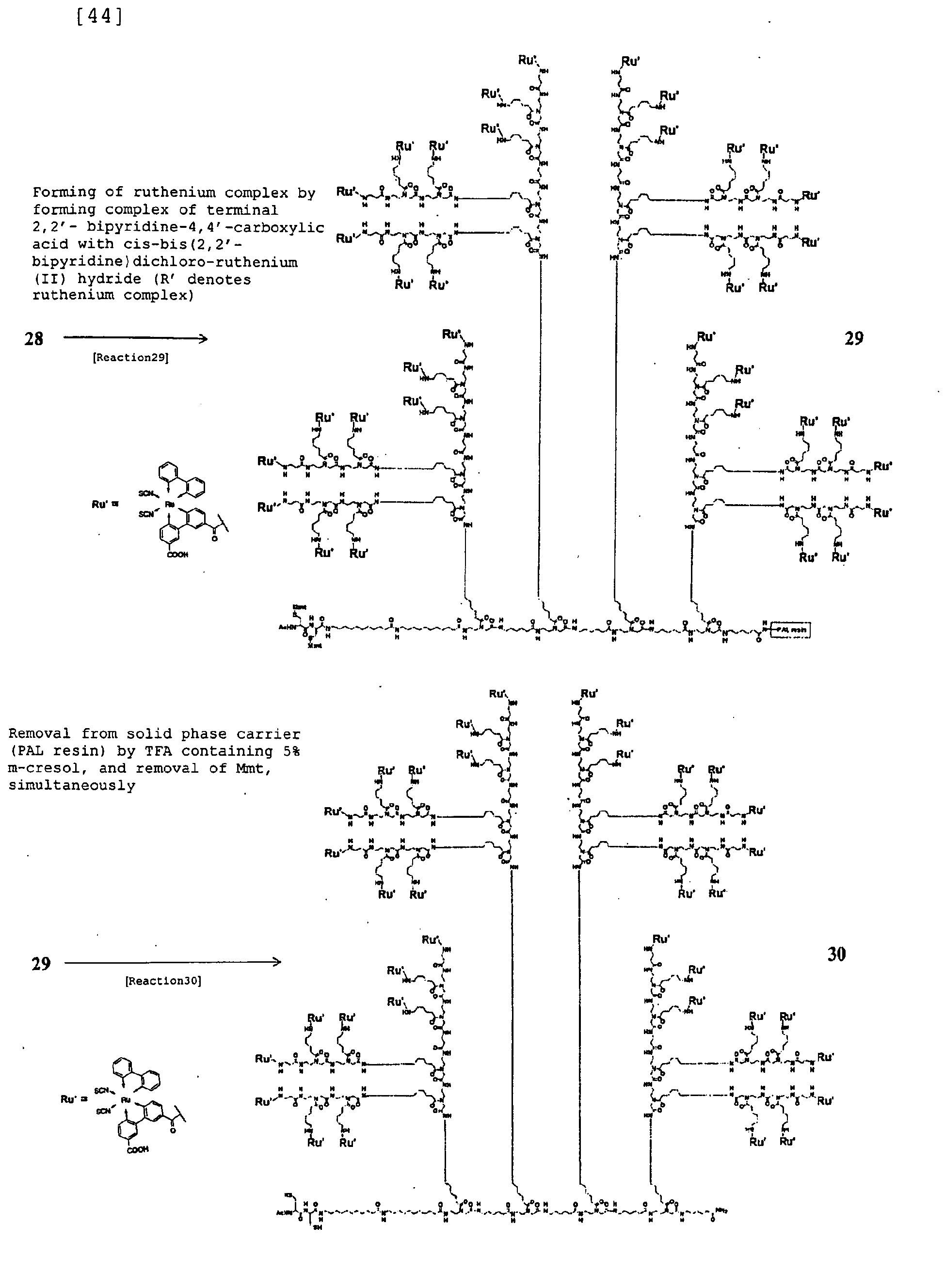

The specific details of the reactions 20-30 are as follows.

### [Reaction 20] Synthesis of the compound shown by the formula 20:

178 mg of the compound shown by the formula 8 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 26.1 mg of Fmoc-Cys(Mmt)-OH, 16.1 mg of HATU and 15 µl of DIEA had been dissolved. Next, the NMP solution was removed by filtration, and the remaining solid phase resin was washed with DMF and methylene chloride (the dehydration condensation of free amino groups with Fmoc-Cys(Mmt)-OH was confirmed by the ninhydrin test).
This procedure was repeated once on the compound thus obtained, and the compound shown by the formula 20 was obtained (the progress of each reaction was confirmed by the ninhydrin test).

### [Reaction 21] Synthesis of the compound shown by the formula 21:

The compound shown by the formula 20 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in a mixed solution of 40 µl of anhydrous acetic acid, 1 ml of piperidine and 1 ml of DMF solution. Next, the reaction solution was removed by filtration, and the remaining solid phase resin was washed with DMF and methylene chloride, and the compound shown by the formula 21 was obtained (the capping of free amino groups was confirmed by the ninhydrin test).

### [Reaction 22] Synthesis of the compound shown by the formula 22:

The compound shown by the formula 21 was treated for 3 minutes with 1 ml of a methylene chloride solution of PhSiH₃ (174.6 µl), 19.6 mg of Pd(PPh₃)₄ was added, and the mixture stirred for 30 minutes. The reaction reagent was removed by filtration, the treated solid phase carrier was washed with methylene chloride, 10% dioxane aqueous solution, DMF, and 1% N,N'-diethyldithiocarbamic acid DMF solution, and a compound having a free amino group was obtained (the removal of the Fmoc group was confirmed by the ninhydrin test).
The compound obtained was stirred at room temperature for 30 minutes in 2 ml of a NMP solution in which 91.2 mg of the compound (D1), 64.6 mg of HATU and 60 µl of DIEA had been dissolved.
Next, the NMP solution was removed by filtration, the remaining solid phase resin was washed with DMF and methylene chloride, and the compound shown by the formula 22 was obtained (the dehydration condensation of free amino groups with the compound (D1) was confirmed by the ninhydrin test).

### [Reaction 23] Synthesis of the compound shown by the formula 23:

The compound shown by the formula 22 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test).
The compound obtained was stirred at room temperature for 30 minutes in 2 ml of a NMP solution in which 91.2 mg of the compound (D1), 64.6 mg of HATU and 60 µl of DIEA had been dissolved.
Next, the NMP solution was removed by filtration, the remaining solid phase resin was washed with DMF and methylene chloride, and the compound shown by the formula 23 was obtained (the dehydration condensation of free amino groups with the compound (D1) was confirmed by the ninhydrin test).

### [Reaction 24] Synthesis of the compound shown by the formula 24:

The compound shown by the formula 23 was stirred at room temperature for 5 minutes in 5 ml of 20% piperidine DMF solution. The reaction solution was removed by filtration, and the remaining solid phase resin was washed with DMF and methylene chloride, and 23' was obtained (the removal of the Fmoc group was confirmed by the ninhydrin test). 23' was stirred at room temperature for 30 minutes in 2 ml of a NMP solution in which 29.4 mg of Aloc-C₂-OH, 64.6 mg of HATU and 60 µl of DIEA had been dissolved. Next, the NMP solution was removed by filtration, the remaining solid phase resin was washed with DMF and methylene chloride, and the compound shown by the formula 24 was obtained (the dehydration condensation of free amino groups with Aloc-C₂-OH was confirmed by the ninhydrin test).

### [Reaction 25] Synthesis of the compound shown by the formula 25:

The compound shown by the formula 24 was treated for 3 minutes with 1 ml of a methylene chloride solution of PhSiH₃ (phenylsilane) (523.8 µl), Pd(PPh₃)₄ (tetrakis(triphenylphospine) palladium (O)) (58.8 mg) was added, and the mixture stirred for 30 minutes. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Fmoc group was confirmed by the ninhydrin test).
The compound obtained was stirred at room temperature for 30 minutes in 2 ml of a NMP solution in which 273.7 mg of the compound (D1), 193.7 mg of HATU and 180 µl of DIEA had been dissolved.
Next, the NMP solution was removed by filtration, and the remaining solid phase resin was washed with DMF and methylene chloride (the dehydration condensation of free amino groups with the compound (D1) was confirmed by the ninhydrin test). Next, the compound obtained was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test).
Next, the compound obtained was stirred at room temperature for 30 minutes in 2 ml of a NMP solution in which 273.7 mg of the compound (D1), 193.7 mg of HATU and 180 µl of DIEA had been dissolved.
Next, the NMP solution was removed by filtration, and the remaining solid phase resin was washed with DMF and methylene chloride (the dehydration condensation of free amino groups with the compound (D1) was confirmed by the ninhydrin test). The compound obtained was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 88.2 mg of Aloc-C₂-OH, 193.7 mg of HATU and 180 µl of DIEA had been dissolved. Next, the NMP solution was removed by filtration, the remaining solid phase resin was washed with DMF and methylene chloride, and the compound shown by the formula 25 (209 mg) was obtained (the dehydration condensation of free amino groups with Aloc-C₂-OH was confirmed by the ninhydrin test).

### [Reaction 26] Synthesis of the compound shown by the formula 26:

30 mg of the compound shown by the formula 25 was stirred for 3 minutes in 1 ml of a methylene chloride solution of PhSiH₃ (216 µl), Pd(PPh₃)₄ (16.3 mg) was added, and the mixture stirred for 30 minutes. Next, the reaction solution was removed by filtration, the remaining solid phase resin was washed with methylene chloride, 10% dioxane aqueous solution, DMF, and 1% N,N'-diethyldithiocarbamic acid DMF solution, and 25' having a free amino group was obtained (the removal of the Fmoc group was confirmed by the ninhydrin test). 25' was stirred at room temperature for 16 hours in NMP solution (1 ml) containing coumarin-3-carboxylic acid (20.5 mg), HATU (41 mg) and DIEA (100 µl). Next, the NMP solution was removed by filtration, the remaining solid phase resin was washed with DMF and methylene chloride, and the compound shown by the formula 26 was obtained (the dehydration condensation of free amino groups with coumarin-3-carboxylic acid was confirmed by the ninhydrin test).

### [Reaction 27] Synthesis of the compound shown by the formula 27:

The compound shown by the formula 26 was stirred away from light for 2 hours in 0.5ml of TFA solution (TFA solution of 5% m-cresol). Next, the TFA solution was recovered by filtration, and the remaining solid phase resin was washed by 0.1 ml of TFA. The filtrate was dripped into cold diethyl ether (10ml), and the precipitate was collected by centrifugal separation. The residue was dissolved in a small amount of methanol, diethyl ether was added to separate it out, and recovery by centrifugal separation was performed twice. Residual ether was removed, and after drying, 2.0 mg of the compound shown by the formula 27 was obtained.

### [Reaction 28] Synthesis of the compound shown by the formula 28:

30 mg of the compound shown by the formula 25 was stirred for 3 minutes in 1 ml of a methylene chloride solution of PhSiH₃ (216 µl), Pd(PPh₃)₄ was added, and the mixture stirred for 30 minutes. Next, the reaction solution was removed by filtration, the remaining solid phase carrier was washed with methylene chloride, 10% dioxane aqueous solution, DMF, and 1% N,N'-diethyldithiocarbamic acid DMF solution, and 25' having a free amino group was obtained (the removal of the Fmoc group was confirmed by the ninhydrin test). 25' was stirred at room temperature for 16 hours in 1 ml of a NMP solution containing 2,2'-bipyridine-4,4'-dicarboxylic acid (bpy) (79 mg), HATU (41 mg) and DIEA (170 µl). Next, the NMP solution was removed by filtration,
the remaining solid phase resin was washed with DMF and methylene chloride, and the compound shown by the formula 28 was obtained (the dehydration condensation of free amino groups with 2,2'-bipyridine-4,4'-dicarboxylic acid was confirmed by the ninhydrin test).

### [Reaction 29] Synthesis of the compound shown by the formula 29:

30 mg (2 µmol (72 µmol: free amino groups), 1.0 eq) of the compound shown by the formula 28 was introduced into an eggplant flask, mixed with 30 ml of an ethanol aqueous solution (ethanol:distilled water =1:7), cis-bis (2,2'-bipyridine) dichloro-ruthenium (II) hydride (181.5 mg, 375 µmol, 5.0 eq) was added, the mixture heated on an oil bath (about 80°) using a reflux condenser, and stirred for 16 hours. The reaction solution was removed by filtration, the residual solid phase resin was washed with 50% methanol aqueous solution, DMF and methylene chloride, and the compound shown by the formula 29 was obtained.

### [Reaction 30] Synthesis of the compound shown by the formula 30:

The compound shown by the formula 29 was stirred at room temperature for 1.5 hours away from light in 0.3 ml of a TFA solution (TFA solution of 5% m-cresol), the TFA solution was recovered by filtration, and the residual solid phase resin was washed with 0.2 ml TFA. The filtrate was dripped into cold diethyl ether (10 ml), and the precipitate was recovered by centrifugation. The residue was dissolved in a small amount of methanol, diethyl ether was added to separate it out, and recovery by centrifugal separation was performed 3 times. Residual ether was removed, and after drying, 0.6 mg of the compound shown by the formula 30 was obtained.

### Third embodiment

The compound shown by the general formula 42 or 44 was manufactured according to the following reaction sequence.

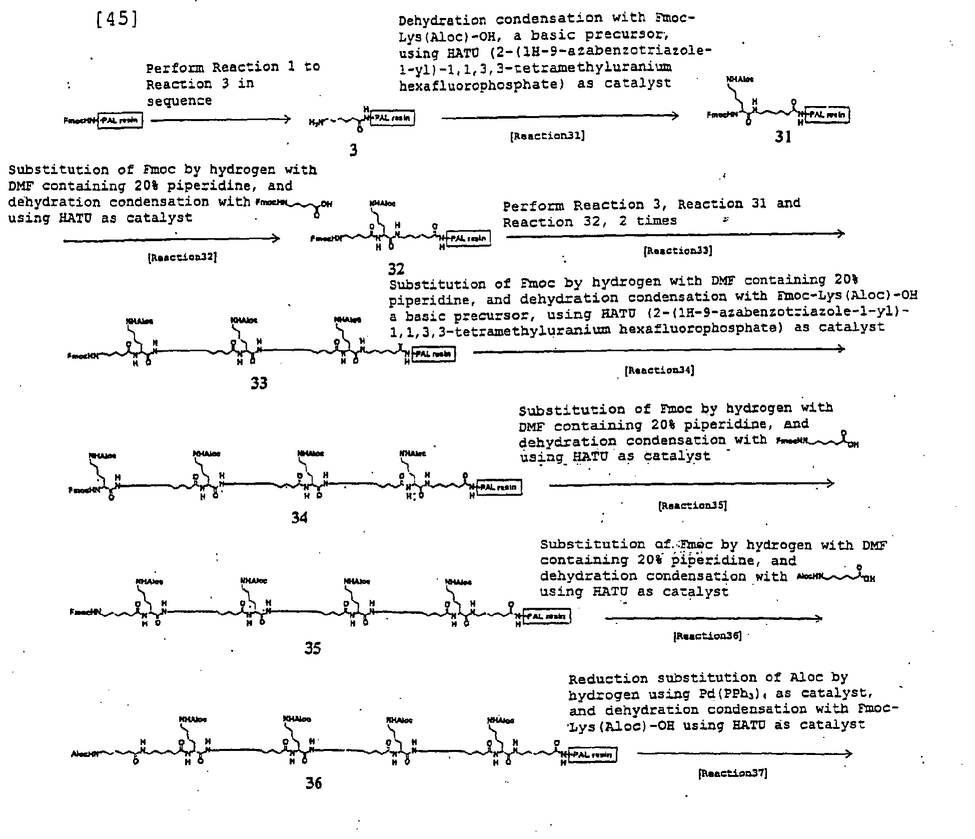

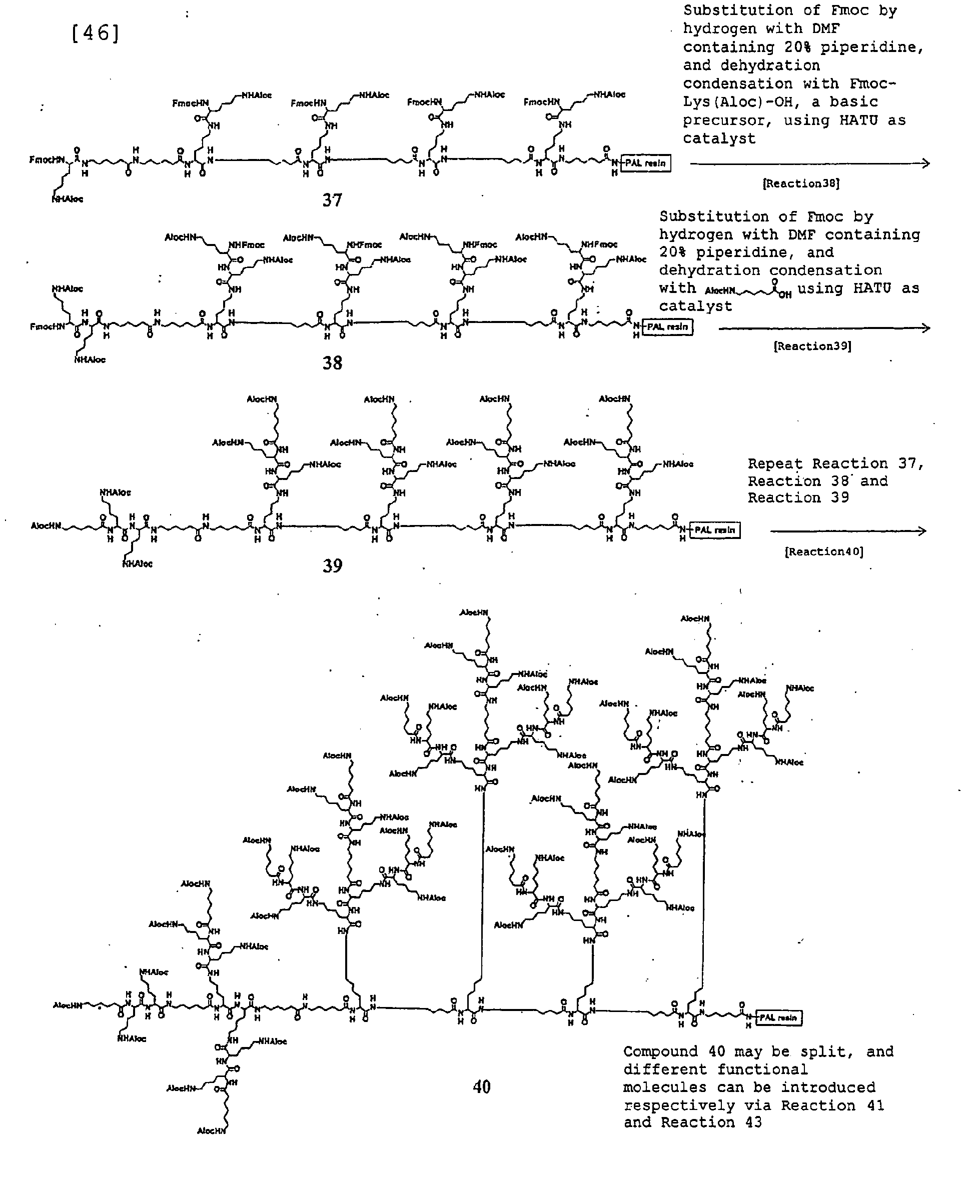

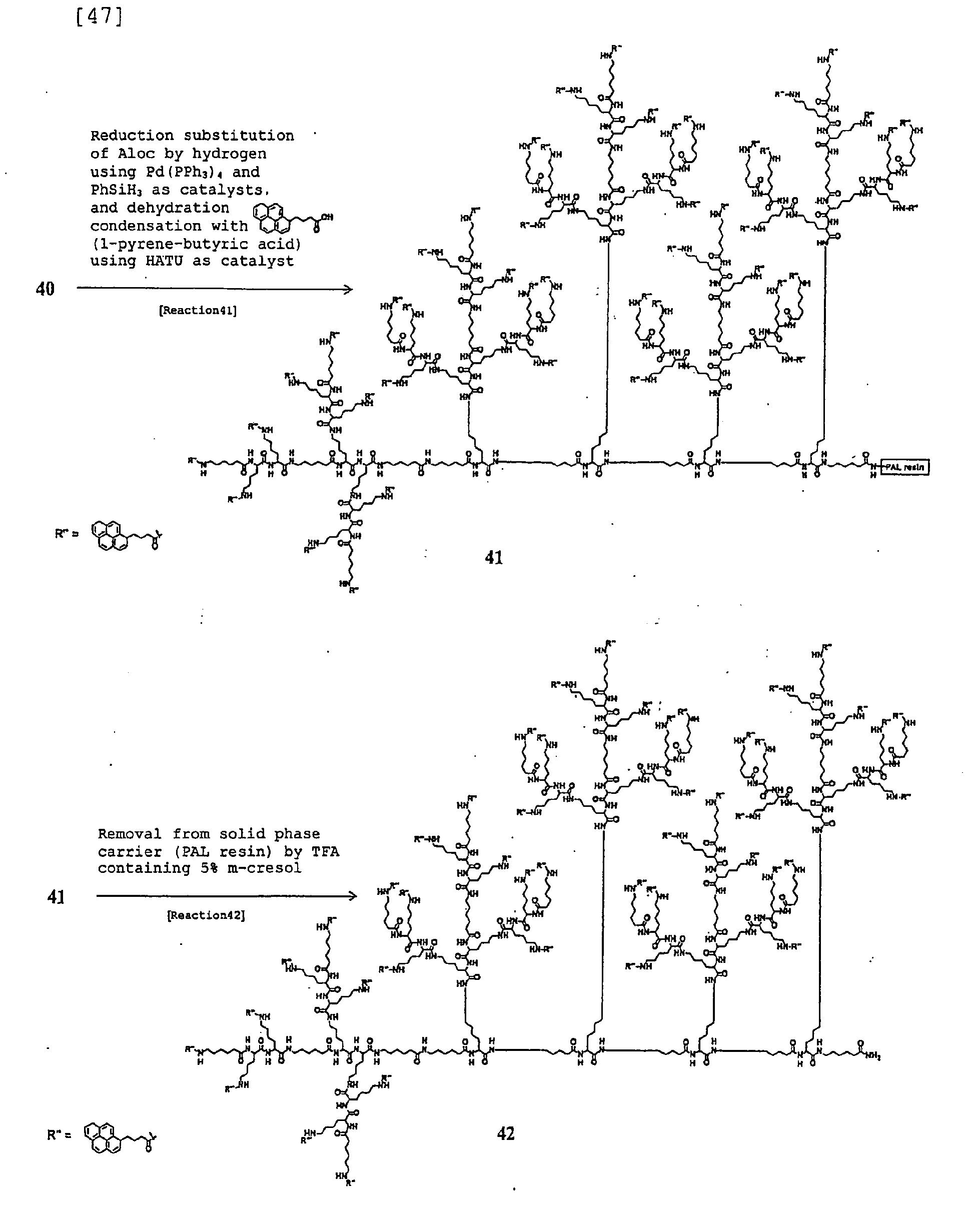

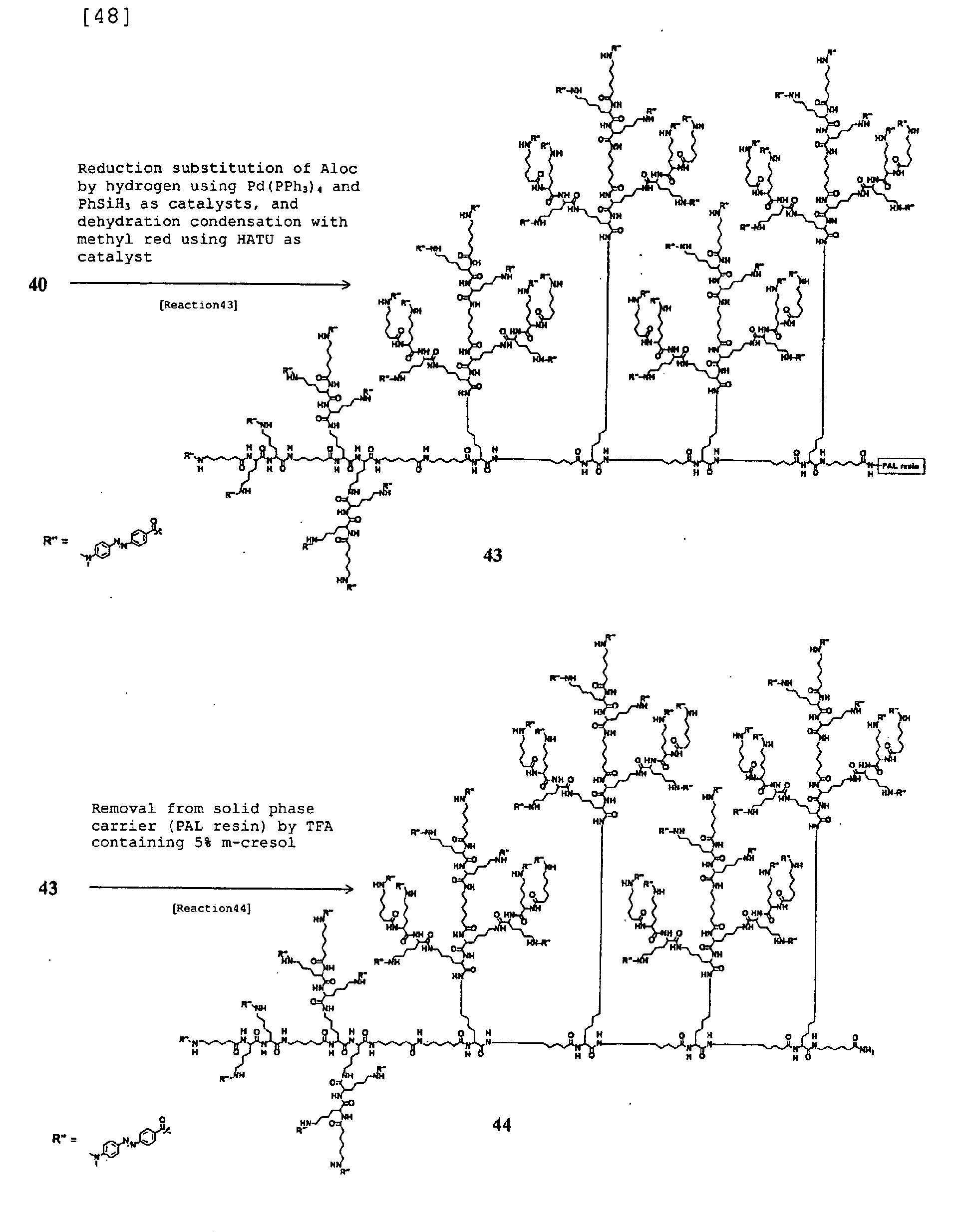

The specific details of the reactions 31-44 are as follows.

### [Reaction 31] Synthesis of the compound shown by the formula 31:

The compound shown by the formula 3 was stirred at room temperature for 30 minutes in 2 ml of a NMP solution in which 19.2 mg of Fmoc-Lys(Aloc)-OH, 19.5 mg of HATU and 12 µl of DIEA had been dissolved. Next, the NMP solution was removed by filtration, the remaining solid phase resin was washed with DMF and methylene chloride, and the compound shown by the formula 31 was obtained (the dehydration condensation of free amino groups with Fmoc-Lys(Aloc)-OH was confirmed by the ninhydrin test).

### [Reaction 32] Synthesis of the compound shown by the formula 32:

The compound shown by the formula 31 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 14.4 mg of Fmoc-C₄-OH, 19.5 mg of HATU and 12 µl of DIEA had been dissolved. Next, the NMP solution was removed by filtration, the remaining solid phase resin was washed with DMF and methylene chloride, and the compound shown by the formula 32 was obtained (the dehydration condensation of free amino groups with Fmoc-C₄-OH was confirmed by the ninhydrin test).

### [Reaction 33] Synthesis of the compound shown by the formula 33:

The reaction 3, reaction 31 and reaction 32 were performed twice in the same way on the compound shown by the formula 32, and the compound shown by the formula 33 was obtained (the progress of each reaction was confirmed by the ninhydrin test).

### [Reaction 34] Synthesis of the compound shown by the formula 34:

The compound shown by the formula 33 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 19.2 mg of Fmoc-Lys(Aloc)-OH, 19.5 mg of HATU and 12 µl of DIEA had been dissolved. Next, the NMP solution was removed by filtration, the remaining solid phase resin was washed with DMF and methylene chloride, and the compound shown by the formula 34 was obtained (the dehydration condensation of free amino groups with Fmoc-Lys(Aloc)-OH was confirmed by the ninhydrin test).

### [Reaction 35] Synthesis of the compound shown by the formula 35:

The compound shown by the formula 34 was stirred at room temperature for 5 minutes in 5 ml of 20% piperidine DMF solution. Next, the reaction solution was removed by filtration, the remaining solid phase resin was washed with DMF and methylene chloride, and 34' was obtained (the removal of the Fmoc group was confirmed by the ninhydrin test). 34' was stirred at room temperature for 30 minutes in 2 ml of a NMP solution in which 15 mg of Emoc-C₅-OH, 19.5 mg of HATU and 12 µl of DIEA had been dissolved.
Next, the NMP solution was removed by filtration, the remaining solid phase resin was washed with DMF and methylene chloride, and the compound shown by the formula 35 was obtained (the dehydration condensation of free amino groups with Fmoc-C₅-OH was confirmed by the ninhydrin test).

### [Reaction 36] Synthesis of the compound shown by the formula 36:

The compound shown by the formula 35 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). Next, the compound obtained was stirred at room temperature for 30 minutes in 2 ml of a NMP solution in which 9.1 mg of Aloc-C₅-OH, 19.5 mg of HATU and 12 µl of DIEA had been dissolved. Next, the NMP solution was removed by filtration, the remaining solid phase resin was washed with DMF and methylene chloride, and the compound shown by the formula 36 was obtained (the dehydration condensation of free amino groups with Aloc-C₅-OH was confirmed by the ninhydrin test).

### [Reaction 37] Synthesis of the compound shown by the formula 37:

The compound shown by the formula 36 was stirred at room temperature for 2 hours in 1.85 ml of a chloroform solution in which 98.1 mg of Pd(PPh₃)₄ (tetrakis(triphenylphospine) palladium (O)), 100 µl of acetic acid, and 50 µl of NMM (N-methylmorpholine) had been dissolved. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound obtained was stirred at room temperature for 30 minutes in 2 ml of a NMP solution in which 96 mg of Fmoc-Lys(Aloc)-OH, 98 mg of HATU and 60 µl of DIEA had been dissolved. Next, the NMP solution was removed by filtration, the remaining solid phase resin was washed with DMF and methylene chloride, and the compound shown by the formula 37 was obtained (the dehydration condensation of free amino groups with Fmoc-Lys(Aloc)-OH was confirmed by the ninhydrin test).

### [Reaction 38] Synthesis of the compound shown by the formula 38:

The compound shown by the formula 37 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 96 mg of Fmoc-Lys(Aloc)-OH, 98 mg of HATU and 60 µl of DIEA had been dissolved. Next, the NMP solution was removed by filtration, the remaining solid phase resin was washed with DMF and methylene chloride, and the compound shown by the formula 38 was obtained (the dehydration condensation of free amino groups with Fmoc-Lys(Aloc)-OH was confirmed by the ninhydrin test).

### [Reaction 39] Synthesis of the compound shown by the formula 39:

The compound shown by the formula 38 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 46 mg of Aloc-C₅-OH, 98 mg of HATU and 60 µl of DIEA had been dissolved. Next, the NMP solution was removed by filtration, the remaining solid phase resin was washed with DMF and methylene chloride, and the compound shown by the formula 39 was obtained (the dehydration condensation of free amino groups with Aloc-C₅-OH was confirmed by the ninhydrin test).

### [Reaction 40] Synthesis of the compound shown by the formula 40:

The compound shown by the formula 39 was stirred at room temperature for 2 hours in 3.7 ml of a chloroform solution in which 196.1 mg of Pd(PPh₃)₄ (tetrakis(triphenylphospine) palladium (O)), 200 µl of acetic acid, and 100 µl of NMM (N-methylmorpholine) had been dissolved. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Fmoc group was confirmed by the ninhydrin test). The compound obtained was stirred at room temperature for 30 minutes in 2 ml of a NMP solution in which 288 mg of Fmoc-Lys(Aloc)-OH, 292 mg of HATU and 180 µl of DIEA had been dissolved. Next, the NMP solution was removed by filtration, and the remaining solid phase resin was washed with DMF and methylene chloride (the dehydration condensation of free amino groups with Fmoc-Lys(Aloc)-OH was confirmed by the ninhydrin test). After washing, the compound was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). Further, the compound obtained was stirred at room temperature for 30 minutes in 3 ml of a NMP solution in which 288 mg of Fmoc-Lys(Aloc)-OH, 292 mg of HATU and 180 µl of DIEA had been dissolved. Next, the NMP solution was removed by filtration, and the remaining solid phase resin was washed with DMF and methylene chloride (the dehydration condensation of free amino groups with Fmoc-Lys(Aloc)-OH was confirmed by the ninhydrin test). The compound obtained was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test).
After washing, the compound obtained was stirred at room temperature for 30 minutes in 3 ml of a NMP solution in which 137 mg of Aloc-C₅-OH, 292 mg of HATU and 180 µl of DIEA had been dissolved. Next, the NMP solution was removed by filtration, the remaining solid phase resin was washed with DMF and methylene chloride, and the compound shown by the formula 40 was obtained (the dehydration condensation of free amino groups with Aloc-C₅-OH was confirmed by the ninhydrin test).

### [Reaction 41] Synthesis of the compound shown by the formula 41:

The compound shown by the formula 40 (30 mg) was treated for 3 minutes with 2.5 ml of a methylene chloride solution of PhSiH₃ (phenylsilane) (216 µl), Pd(PPh₃)₄ (tetrakis(triphenylphospine) palladium (O)) (16.3 mg) was added, and the mixture stirred for 30 minutes. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound obtained was stirred at room temperature for 16 hours in 2 ml of a NMP solution in which 101 mg of pyrene-1-butyric acid, 134 mg of HATU and 61 µl of DIEA had been dissolved. Next, the NMP solution was removed by filtration, the remaining solid phase resin was washed with DMF and methylene chloride, and the compound shown by the formula 41 was obtained (the dehydration condensation of free amino groups with pyrene-1-butyric acid was confirmed by the ninhydrin test).

### [Reaction 42] Synthesis of the compound shown by the formula 42:

The compound shown by the formula 41 was stirred away from light for 1.5 hours in 0.3 ml of TFA solution (TFA solution of 5% m-cresol). Next, the TFA solution was recovered by filtration, and the remaining solid phase resin was washed by 0.1 ml of TFA. The filtrate was dissolved in diethyl ether, and washed with 5% aqueous sodium bicarbonate and distilled water. Next, the diethyl ether was distilled off, the residue dried, and 3.0 mg of the compound shown by the formula 42 was obtained.

### [Reaction 43] Synthesis of the compound shown by the formula 43:

The compound shown by the formula 40 was treated for 3 minutes with 2.5 ml of a methylene chloride solution of PhSiH₃ (phenylsilane) (216 µl), Pd(PPh₃)₄ (tetrakis(triphenylphospine) palladium (O)) (16.3 mg) was added, and the mixture stirred for 30 minutes. The reaction reagent was removed by filtration, the treated solid phase carrier was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound thus obtained was stirred at room temperature for 16 hours in 2 ml of a NMP solution in which 95 mg of o-methyl red), 134 mg of HATU and 61 µl of DIEA had been dissolved. Next, the NMP solution was removed by filtration, the remaining solid phase resin was washed with DMF and methylene chloride, and the compound shown by the formula 43 was obtained (the dehydration condensation of free amino groups with o-methyl red was confirmed by the ninhydrin test).

### [Reaction 44] Synthesis of the compound shown by the formula 44:

The compound shown by the formula 43 was stirred away from light for 1.5 hours in 0.3 ml of TFA solution (TFA solution of 5% m-cresol). Next, the TFA solution was recovered by filtration, and the remaining solid phase resin was washed by 0.1 ml of TFA. The filtrate was dissolved in diethyl ether, and washed with 5% aqueous sodium bicarbonate and distilled water. Next, the diethyl ether was distilled off, the residue dried, and 1.0 mg of the compound shown by the formula 44 was obtained.

### Fourth embodiment

The compounds shown by the general formulae 55 or 57 were likewise obtained as follows.

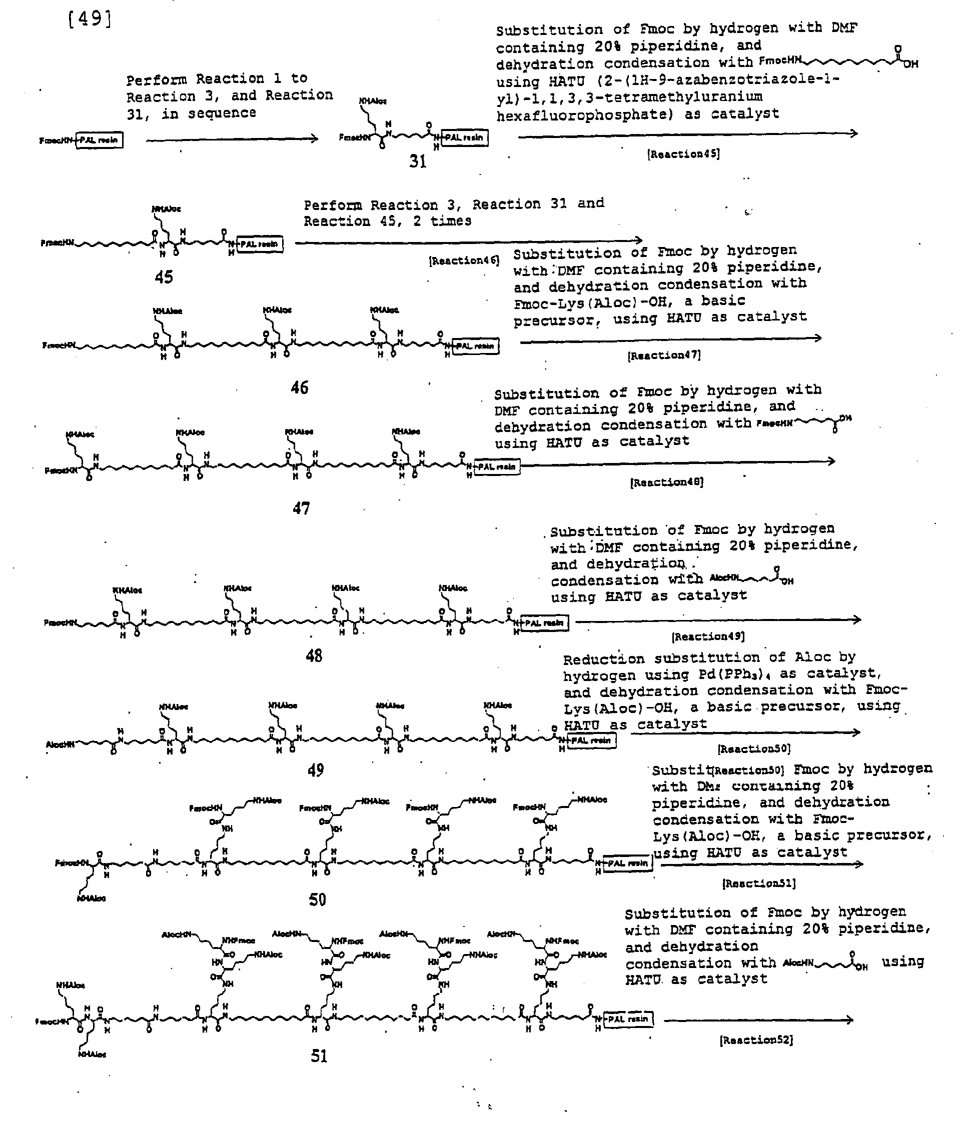

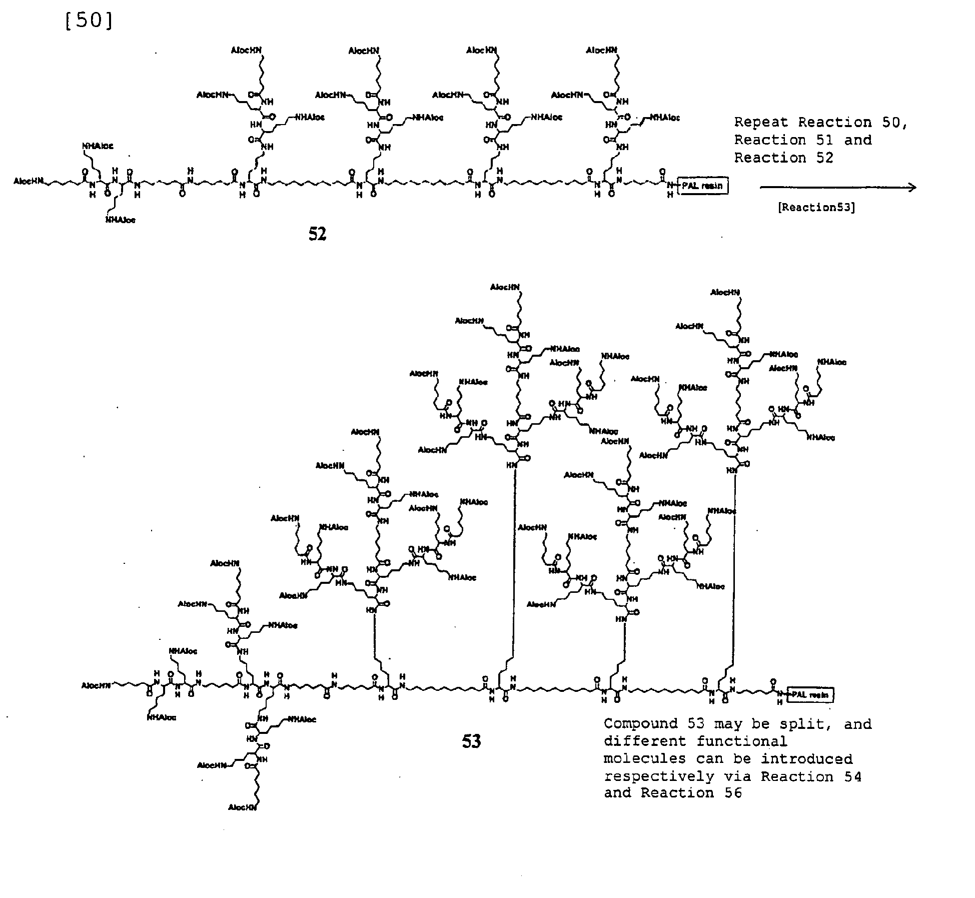

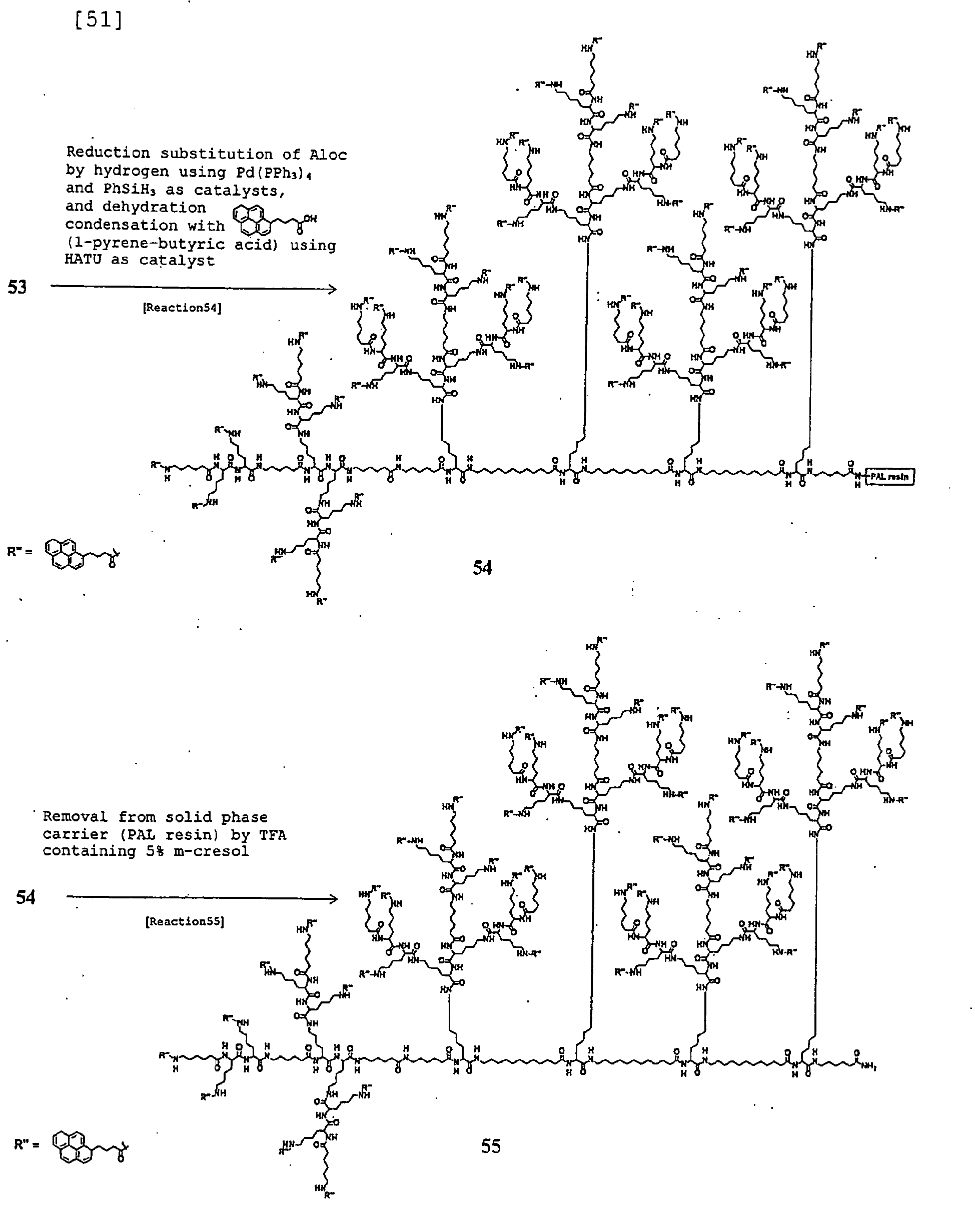

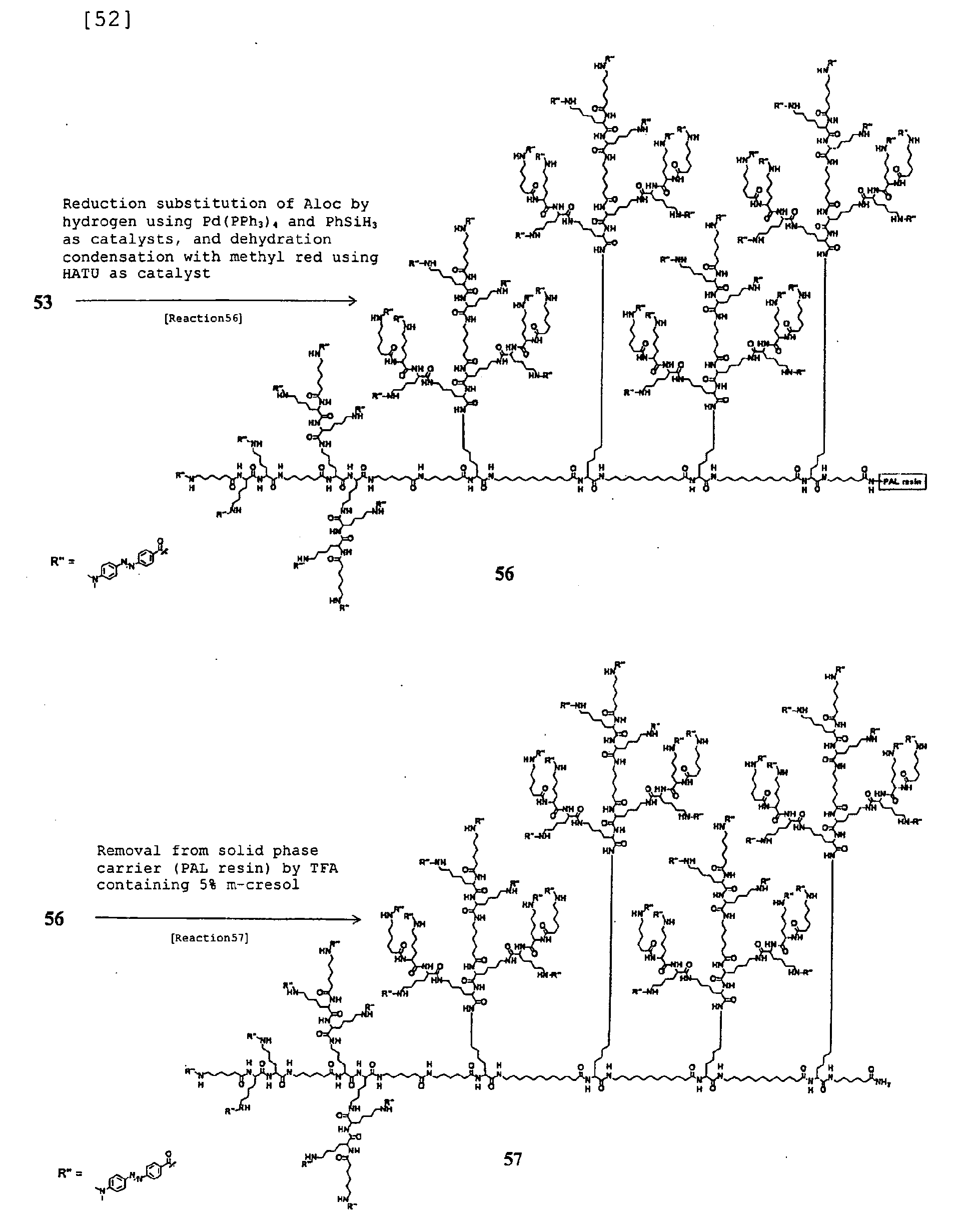

The specific details of the reactions 45-57 are as follows.

### [Reaction 45] Synthesis of the compound shown by the formula 45:

The compound shown by the formula 31 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 18.5 mg of Fmoc-C₁₁-OH, 19.5 mg of HATU and 12 µl of DIEA had been dissolved. Next, the NMP solution was removed by filtration, the remaining solid phase resin was washed with DMF and methylene chloride, and the compound shown by the formula 45 was obtained (the dehydration condensation of free amino groups with Fmoc-C₁₁-OH was confirmed by the ninhydrin test).

### [Reaction 46] Synthesis of the compound shown by the formula 46:

The reaction 3, reaction 31 and reaction 45 were performed twice in the same way on the compound shown by the formula 45, and the compound shown by the formula 46 was obtained (the progress of each reaction was confirmed by the ninhydrin test).

### [Reaction 47] Synthesis of the compound shown by the formula 47:

The compound shown by the formula 46 was stirred at room temperature for 5 minutes in 5 ml of 20% piperidine DMF solution. Next, the reaction solution was removed by filtration, the remaining solid phase resin was washed with DMF and methylene chloride, and 46' was obtained (the removal of the Fmoc group was confirmed by the ninhydrin test). 46' was stirred at room temperature for 30 minutes in 2 ml of a NMP solution in which 19.2 mg of Fmoc-Lys(Aloc)-OH, 19.5 mg of HATU and 12 µl of DIEA had been dissolved. Next, the NMP solution was removed by filtration, the remaining solid phase resin was washed with DMF and methylene chloride, and the compound shown by the formula 47 was obtained (the dehydration condensation of free amino groups with Fmoc-Lys(Aloc)-OH was confirmed by the ninhydrin test).

### [Reaction 48] Synthesis of the compound shown by the formula 48:

The compound shown by the formula 47 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 15 mg of Fmoc-C₅-OH, 19.5 mg of HATU and 12 µl of DIEA had been dissolved. Next, the NMP solution was removed by filtration, the remaining solid phase resin was washed with DMF and methylene chloride, and the compound shown by the formula 48 was obtained (the dehydration condensation of free amino groups with FmoC-C₅-OH was confirmed by the ninhydrin test).

### [Reaction 49] Synthesis of the compound shown by the formula 49:

The compound shown by the formula 48 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 2 ml of a NMP solution in which 9.1 mg of Aloc-C₅-OH, 19.5 mg of HATU and 12 µl of DIEA had been dissolved. Next, the NMP solution was removed by filtration, the remaining solid phase resin was washed with DMF and methylene chloride, and the compound shown by the formula 49 was obtained (the dehydration condensation of free amino groups with Aloc-C₅-OH was confirmed by the ninhydrin test).

### [Reaction 50] Synthesis of the compound shown by the formula 50:

The compound shown by the formula 49 was stirred at room temperature for 2 hours in 1.85 ml of a chloroform solution
in which 98.1 mg of Pd(PPh₃)₄ (tetrakis(triphenylphospine) palladium (O)), 100 µl of acetic acid, and 50 µl of NMM (N-methylmorpholine) had been dissolved. The reaction solution was removed by filtration, the treated solid phase carrier was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound obtained was stirred at room temperature for 30 minutes in 2 ml of a NMP solution in which 96 mg of Fmoc-Lys(Aloc)-OH, 98 mg of HATU and 60 µl of DIEA had been dissolved. Next, the NMP solution was removed by filtration, the remaining solid phase resin was washed with DMF and methylene chloride, and the compound shown by the formula 50 was obtained (the dehydration condensation of free amino groups with Fmoc-Lys(Aloc)-OH was confirmed by the ninhydrin test).

### [Reaction 51] Synthesis of the compound shown by the formula 51:

The compound shown by the formula 50 was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test). The compound obtained was stirred at room temperature for 30 minutes in 2 ml of a NMP solution in which 96 mg of Fmoc-Lys (Aloc)-OH, 98 mg of HATU and 60 µl of DIEA had been dissolved. Next, the NMP solution was removed by filtration, the remaining solid phase resin was washed with DMF and methylene chloride, and the compound shown by the formula 51 was obtained (the dehydration condensation of free amino groups with Fmoc-Lys(Aloc)-OH was confirmed by the ninhydrin test).

### [Reaction 52] Synthesis of the compound shown by the formula 52:

The compound shown by the formula 51 was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test). The compound obtained was stirred at room temperature for 30 minutes in 3 ml of a NMP solution in which 46 mg of Aloc-C₅-OH, 98 mg of HATU and 60 µl of DIEA had been dissolved. Next, the NMP solution was removed by filtration, the remaining solid phase resin was washed with DMF and methylene chloride, and the compound shown by the formula 52 was obtained (the dehydration condensation of free amino groups with Aloc-C₅-OH was confirmed by the ninhydrin test).

### [Reaction 53] Synthesis of the compound shown by the formula 53:

The compound shown by the formula 52 was stirred at room temperature for 2 hours in 3.7 ml of a chloroform solution in which 196.1 mg of Pd(PPh₃)₄ (tetrakis (triphenylphospine) palladium (O)), 200 µl of acetic acid, and 100 µl of NMM (N-methylmorpholine) had been dissolved. The reaction reagent was removed by filtration, the treated solid phase carrier was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound obtained was stirred at room temperature for 30 minutes in 2 ml of a NMP solution in which 288 mg of Fmoc-Lys(Aloc)-OH, 292 mg of HATU and 180 µl of DIEA had been dissolved. Next, the NMP solution was removed by filtration, and the remaining solid phase resin was washed with DMF and methylene chloride (the dehydration condensation of free amino groups with Fmoc-Lys(Aloc)-OH was confirmed by the ninhydrin test). The compound thus obtained was stirred at room temperature for 5 minutes in 5 ml of 20% piperidine DMF solution. The reaction solution was removed by filtration, and the remaining solid phase resin was washed with DMF and methylene chloride (the removal of the Fmoc group was confirmed by the ninhydrin test).
Further, the compound obtained was stirred at room temperature for 30 minutes in 3 ml of a NMP solution in which 288 mg of Fmoc-Lys(Aloc)-OH, 292 mg of HATU and 180 µl of DIEA had been dissolved. Next, the NMP solution was removed by filtration, and the remaining solid phase resin was washed with DMF and methylene chloride (the dehydration condensation of free amino groups with Fmoc-Lys(Aloc)-OH was confirmed by the ninhydrin test). Next, the compound obtained was stirred at room temperature for 5 minutes in 5 ml of 20% piperidine DMF solution. Next, the reaction solution was removed by filtration, and the remaining solid phase resin was washed by DMF and methylene chloride (the removal of the Fmoc group was confirmed by the ninhydrin test).
The compound thus obtained was stirred at room temperature for 30 minutes in 3 ml of a NMP solution in which 137 mg of Aloc-C₅-OH, 292 mg of HATU and 180 µl of DIEA had been dissolved.
Next, the NMP solution was removed by filtration, the remaining solid phase resin was washed with DMF and methylene chloride, and the compound shown by the formula 53 was obtained (the dehydration condensation of free amino groups with Aloc-C₅-OH was confirmed by the ninhydrin test).

### [Reaction 54] Synthesis of the compound shown by the formula 54:

The compound shown by the formula 53 (15 mg) was treated for 3 minutes in 1 ml of a methylene chloride solution of PhSiH₃ (216 µl), Pd(PPh₃)₄ (16.3 mg) was added, and the mixture stirred for 30 minutes. The reaction solution was removed by filtration, the remaining solid phase resin was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound thus obtained was stirred at room temperature for 16 hours in 1 ml of a NMP solution in which 101 mg of pyrene-1-butyric acid, 134 mg of HATU and 61 µl of DIEA had been dissolved. Next, the NMP solution was removed by filtration, the remaining solid phase resin was washed with DMF and methylene chloride, and the compound shown by the formula 54 was obtained (the dehydration condensation of free amino groups with pyrene-1-butyric acid was confirmed by the ninhydrin test).

### [Reaction 55] Synthesis of the compound shown by the formula 55:

The compound shown by the formula 54 was stirred away from light for 1.5 hours in 0.3ml of TFA solution (TFA solution of 5% m-cresol). Next, the TFA solution was recovered by filtration, and the remaining solid phase resin was washed by 0.1 ml of TFA. The filtrate was dissolved in diethyl ether, and washed with 5% sodium bicarbonate aqueous solution and distilled water. Next, diethyl ether was distilled off and after drying, 0.3 mg of the compound shown by the formula 55 was obtained.

### [Reaction 56] Synthesis of the compound shown by the formula 56:

The compound shown by the formula 53 (15 mg) was stirred for 3 minutes in 1 ml of a methylene chloride solution of PhSiH₃ (216 µl), Pd(PPh₃)₄ (16.3 mg) was added, and the mixture stirred for 30 minutes. The reaction reagent was removed by filtration, the treated solid phase carrier was washed with methylene chloride, 10% dioxane aqueous solution, DMF, and 1% N,N'-diethyldithiocarbamic acid DMF solution, and a compound having a free amino group was obtained (the removal of the Aloc group was confirmed by the ninhydrin test). The compound obtained was stirred at room temperature for 16 hours in 1 ml of a NMP solution in which 95 mg of o-methyl red, 134 mg of HATU and 61 µl of DIEA had been dissolved. Next, the NMP solution was removed by filtration, the remaining solid phase resin was washed with DMF and methylene chloride, and the compound shown by the formula 56 was obtained (the dehydration condensation of free amino groups with o-methyl red was confirmed by the ninhydrin test).

### [Reaction 57] Synthesis of the compound shown by the formula 57:

The compound shown by the formula 56 was stirred away from light for 1.5 hours in 0.3 ml of TFA solution (TFA solution of 5% m-cresol). Next, the TFA solution was recovered by filtration, and the remaining solid phase resin was washed by 0.1 ml of TFA. The filtrate was dissolved in diethyl ether, and washed with 5% sodium bicarbonate aqueous solution and distilled water. Next, the diethyl ether was removed, and after drying, 0.7 mg of 57 (LB-PAL-2) was obtained.

### Fifth embodiment

### Manufacture of photoelectrode

Commercial nanocrystal TiO₂ (P-25) was added to nitric acid solution to 30 wt%, to give a TiO₂ dispersion slurry. 1 g of this TiO₂ dispersion slurry, 1.1 g of polyethylene glycol, and 15 µl of triton XL were mixed to give a paste, and this was coated to an area of 1 cm² on conductive glass (FTO glass). This electrically conductive glass was calcinated at 450°C with an electric furnace (Product of AS ONE Corporation, AK0090-010 (Furnance 1300)) for 30 minutes, and a titania coat transparent electrode was produced.

The titania-coated transparent electrode thus obtained was then immersed in an ethanol solution containing the compound shown by the formula 19 at a concentration of 1x10⁴ mol/L at 50°C for 2 days. After immersion, the electrode was dried, and taken as the photoelectrode of a solar cell. The photoelectrode thus manufactured has a deep color of titania as compared with an photoelectrode to which a Ru complex (RuL₂(NCS)₂, L= 2,2'-bipyridine derivative, where NCS is N=C=S used as a ligand) is adsorbed, which was used conventionally. It became clear that the compound shown by the formula 19 had excellent adsorption on titania.

### Sixth embodiment Manufacture of photoelectrode

An photoelectrode was produced under the same conditions as in the fifth embodiment, except that the titania coated transparent electrode was immersed in the ethanol solution containing the compound shown by the formula 19 for 4 days.

### Seventh embodiment Manufacture and performance of solar cell

### 1. Manufacture of Solar Cell

A platinum coating was applied to conductive glass (FTO glass) separately to form a platinum film of 10nm on the conductive glass, and this was used as a counter electrode. The photoelectrode and counter electrode manufactured according to the fifth and sixth embodiments were brought against each other so that the surfaces of their conductive layers were in contact. Next, by running in a fluorine electrolyte between the photoelectrode and a counter electrode, and sealing with an epoxy super glue (Araldite) to manufacture an outer frame, a dye-sensitized solar cell was manufactured.

### 2. Performance of Solar Cell

The current-voltage (I-V curve) characteristic when the dye-sensitized solar cell thus manufactured was optically irradiated at 50 mW/cm² from a tungsten (W) lamp, was measured using a multimeter (MFG45-01), a product of Fluke Corporation, and the photovoltaic conversion efficiency η (%) was calculated.

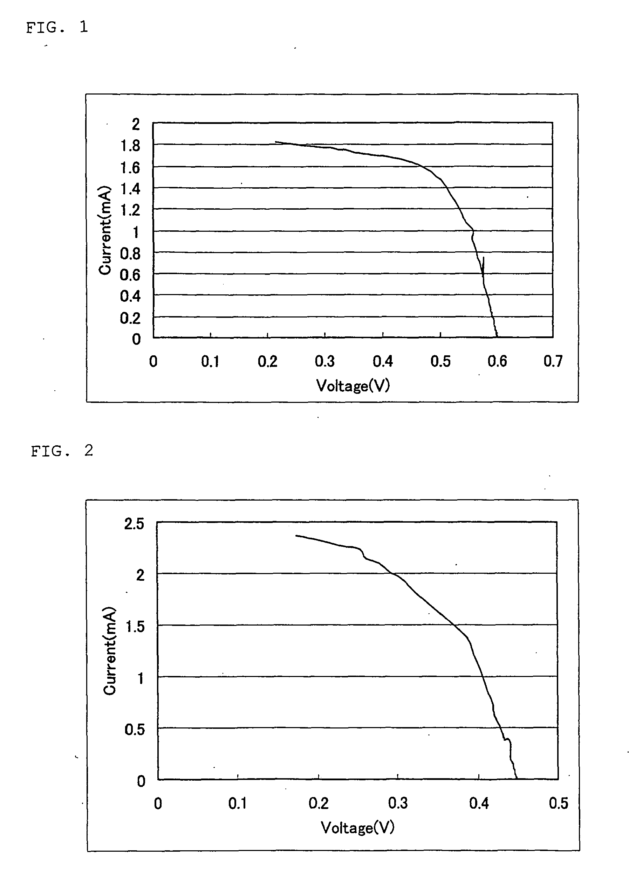

The main results are shown in Table 1, and FIGs. 1 and 2. From this result, it became clear that by using the photoelectrode manufactured in the fifth or sixth embodiments, electrical energy could be obtained with an excellent photovoltaic conversion efficiency.

**[Table 1]**

| Photoelectrode | Max. current (Imax) | Max. voltage (Vmax) | SC Current (Jsc) | Discharge voltage (Voc) | Curve factor (ff) | Photovoltaic conversion efficiency (η) |
|---|---|---|---|---|---|---|
| Embodiment5 | 1.542mA | 0.486V | 1.852mA | 0.6005 V | 0.685 | 1.49% |

### BRIEF DESCRIPTION OF THE DRAWINGS

FIG. 1 is a diagram showing the result of measuring the current-voltage (I-V curve) characteristic using the photoelectrode manufactured according to the fifth embodiment.
FIG. 2 is a diagram showing the result of measuring the current-voltage (I-V curve) characteristic using the photoelectrode manufactured according to the sixth embodiment.

## Claims

1. A compound shown by the following general formula (1): 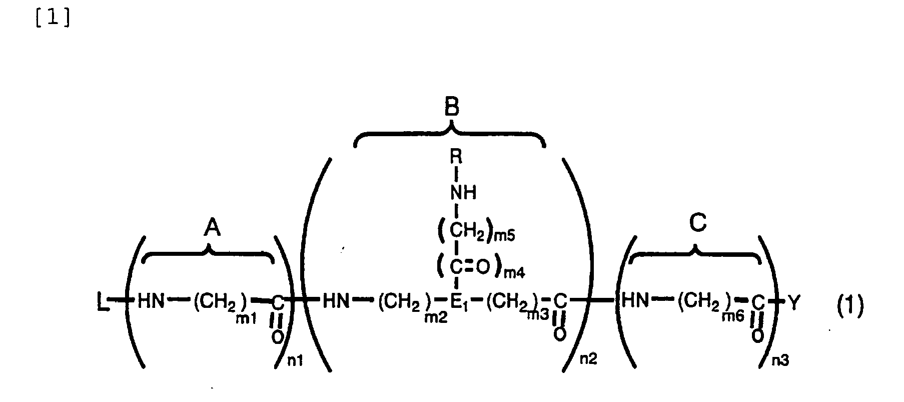 (wherein, in the formula (1), n1 is an integer of 0-10, n2 is an integer of 1-50, and n3 is an integer of 1-10; ml is an integer of 0-100, m2 is an integer of 0-100, m3 is an integer of 0-100, m4 is an integer equal to 0 or 1, m5 is an integer of 0-100, and m6 is an integer of 0-100; Y is a hydroxyl group or an amino group; E1 is N or CH; L is a hydrogen atom or a photosensitizing group; R is a group consisting of one of the basic units D shown by the following general formula (D), or a group having a branched structure wherein a structural unit is repeatedly bonded via a peptide linkage, this structural unit being 1-100 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms. R is bonded via a peptide linkage to a nitrogen atom in the repeating unit B; in R, when two or more of the basic units D are bonded together, these basic units may be identical or different; in R, when two or more of the basic units G are bonded together, these basic units may be identical or different; when n1 is two or more, m1 in the repeating unit A may be identical or different in respective repeating units A; when n2 is 2 or more, m2-m5 and R in the repeating unit B may be identical or different in respective repeating units B; and when n3 is 2 or more, m6 in the repeating unit C may be identical or different in respective repeating units C). 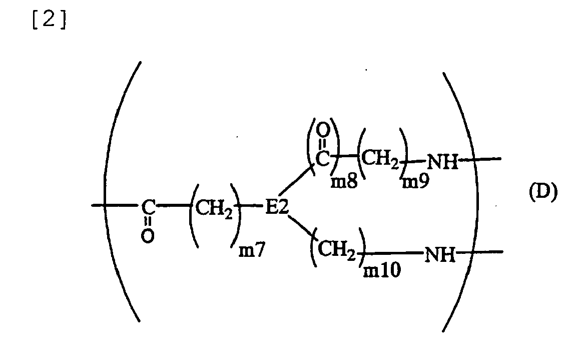 (wherein, in the formula (D), m7 is an integer of 0-100, m8 is an integer equal to 0 or 1, m9 is an integer of 1-100, and m10 is an integer of 0-100;and E2 is N or CH). 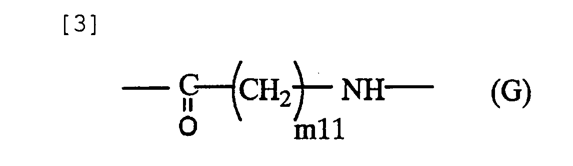 (wherein, in the formula (G), m11 is an integer of 1-100).

2. The compound according to claim 1, wherein R is a group including 1-100 of the basic units D, wherein m7 is 1 or 2, m8 is 1, m9 is an integer of 0-11, m10 is an integer of 1-3, and E2 is N.

3. The compound according to claim 1, wherein R is a group including 1-100 of the basic units D, wherein m7 is 0-2, m8 is 0, m9 is an integer of 0-11, and m10 is 0-2, and E2 is CH.

4. The compound according to claim 1, wherein n1 is an integer of 0-5, n2 is an integer of 1-20, and n3 is an integer of 0-5.

5. The compound according to claim 1, wherein n1 is an integer of 0-2, n2 is an integer of 1-10, and n3 is an integer of 0-2.

6. A method of manufacturing the compound shown by the following general formula (1), including the following steps 1-1 to 1-6. 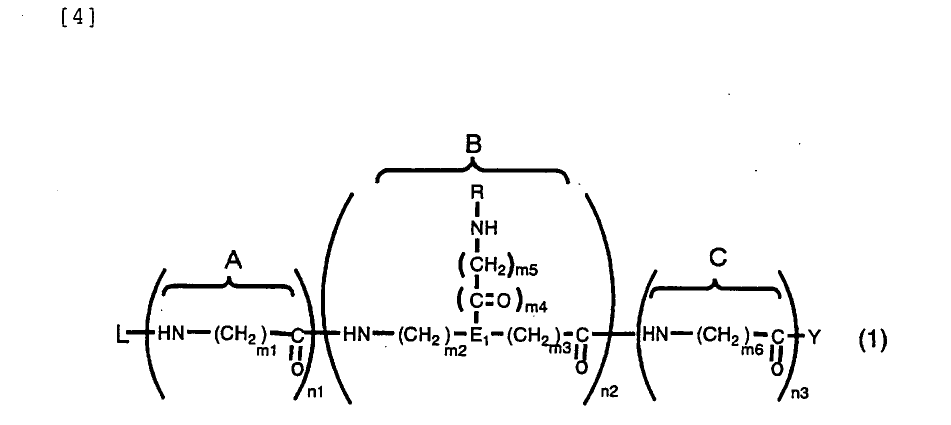 (wherein, in the formula (1), n1 is an integer of 0-10, n2 is an integer of 1-50, and n3 is an integer of 1-10; m1 is an integer of 0-100, m2 is an integer of 0-100, m3 is an integer of 0-100, m4 is an integer equal to 0 or 1, m5 is an integer of 0-100, m6 is an integer of 0-100; Y is a hydroxyl group or an amino group; E1 is N or CH; L is a hydrogen atom or a photosensitizing group; R is a group consisting of one of the basic units D shown by the following general formula (D), or a group having a branched structure wherein a structural unit is repeatedly bonded via a peptide linkage, this structural unit being 1-10 of the basic units D and 1-100 of the basic units G shown by the following general formula (G). The photosensitizing group is bonded to 50% or more of the nitrogen atoms among the nitrogen atoms which do not constitute the peptide linkage of this group, hydrogen atoms being bonded to the remaining nitrogen atoms. R is bonded via a peptide linkage to a nitrogen atom in the repeating unit B; in R, when two or more of the basic units D are bonded together, these basic units may be identical or different; in R, when two or more of the basic units G are bonded together, these basic units may be identical or different; when n1 is two or more, ml in the repeating unit A may be identical or different in respective repeating units A; when n2 is 2 or more, m2-m5 and R in the repeating unit B may be identical or different in respective repeating units B; and when n3 is 2 or more, m6 in the repeating unit C may be identical or different in respective repeating units C). 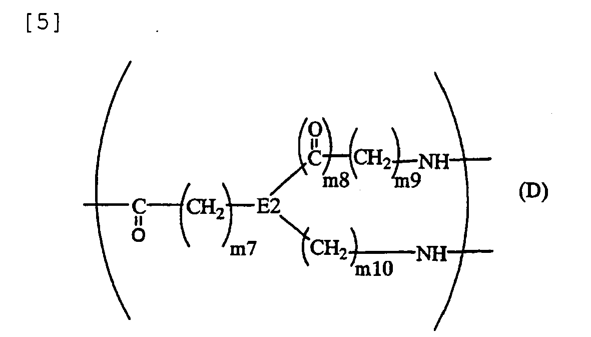 (wherein, in the formula (D), m7 is an integer of 0-100, m8 is an integer equal to 0 or 1, m9 is an integer of 1-100, m10 is an integer of 0-100; and E2 is N or CH). 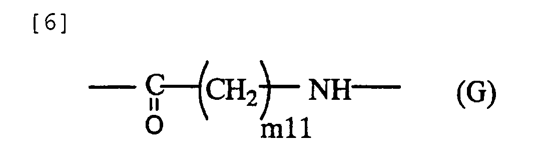 (wherein, in the formula (G), m11 is an integer of 1-100). Step 1-1: The compound shown by the general formula (i): 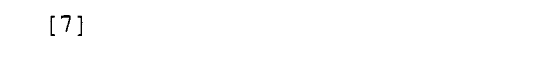 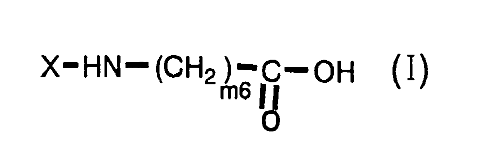 (wherein, in the formula (I), m6 is identical to the above, and X is a protective group),
is obtained by performing a condensation of the compound shown by the general formula (I) with a solid phase resin or a solid phase compound, removing the protective group X of the compound that was condensed with the solid phase resin or solid phase compound, and performing a condensation polymerization of the compound shown by the general formula (I), n3-1 times. 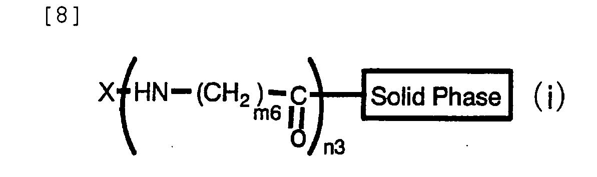 (wherein, in the formula (i), n3, m6, and X are identical to the above, and Solid Phase denotes a solid phase resin or a solid phase compound).
Step 1-2: The compound shown by the general formula (ii) is obtained by performing a condensation reaction of the compound shown by the general formula (II) with the compound shown by the general formula (i), n2 times. 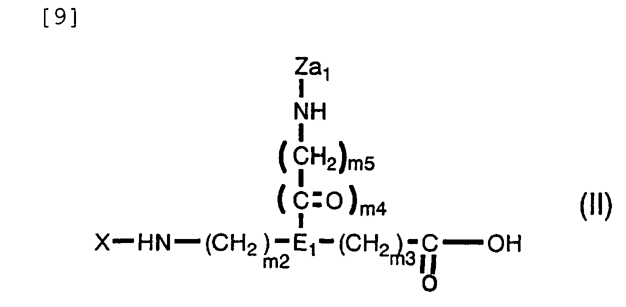 (wherein, in the formula (II), m2-m5, E1 and X are identical to the above, and Za1 is a protective group different from X). 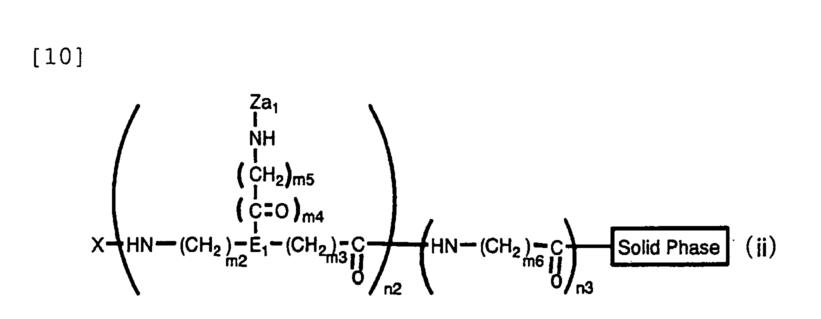 (wherein, in the formula (ii), n2, n3, m2-m6, X, E1, Za1 and Solid Phase are identical to the above).
Step 1-3: The compound shown by the general formula (iii) is obtained by performing a condensation reaction of the compound shown by the general formula (III) with the compound shown by the general formula (ii), n1 times. 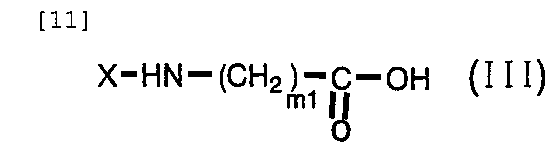 (wherein, in the formula (III), m1 and X are identical to the above). 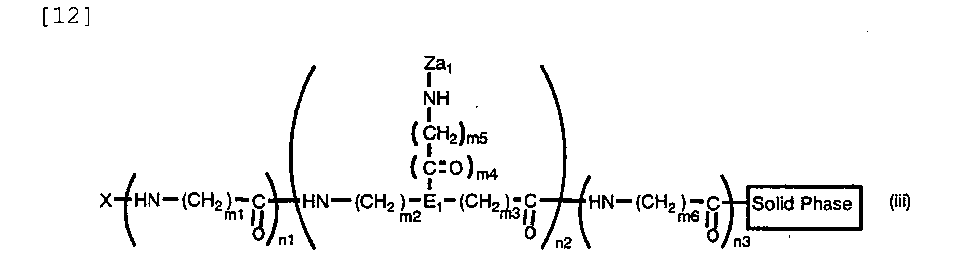 (wherein, in the formula (iii), n1-n3, m1-m6, X, E1, Za1 and Solid Phase are identical to the above).
Step 1-4: The compound shown by the general formula (iv) is obtained by performing a condensation reaction of one of the compounds shown by the following general formula (IV) with the compound shown by the general formula (iii). 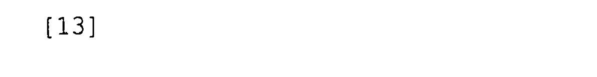 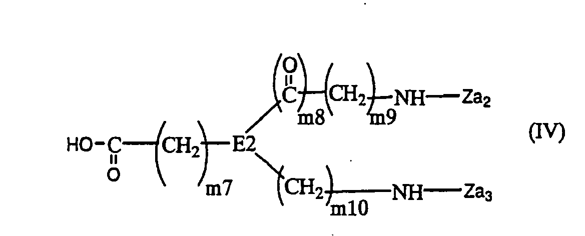 (wherein, in the formula (IV), m7-m10 are identical to the above, Za2 and Za3 are protective groups which may be respectively identical or different, which are different from X, but which may be identical to Za1).
Alternatively, the compound shown by the following general formula (iv) is obtained by performing a condensation reaction of 1-100 of the compounds shown by the general formula (IV) and 1-100 compounds shown by the general formula (V) with the compound shown by the general formula (iii). 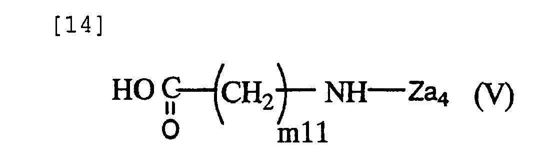 (wherein, in the formula (V), m11 is identical to the above, Za4 is a protective group different from X, and may be identical to Za1, Za2, and Za3). 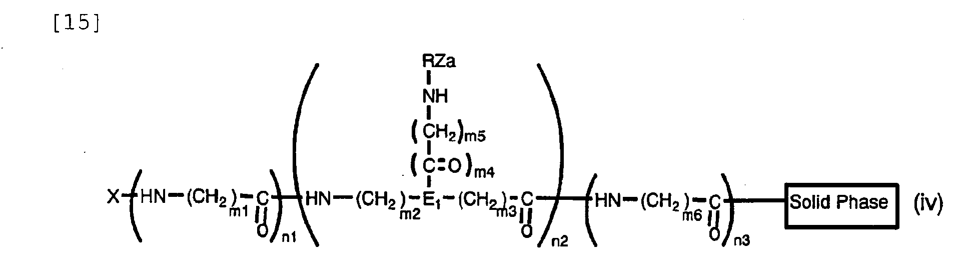 (wherein, in the formula (iv), n1-n3, m1-m6, X, and Solid Phase are identical to the above. Rza is a group having one of the basic units D shown by the following general formula (D), or a group having a branched structure wherein a structural unit is repeatedly bonded via a peptide linkage, this structural unit being 1-100 of the basic units D and 1-100 of the basic units G shown by the following general formula (G), and denotes a group wherein the nitrogen atoms which do not constitute the peptide linkage of this group are protected by the protective groups Za3 and Za4, or the protective groups Za2, Za3, and Za4).
Step 1-5: One, two or more of the protective groups Za2, Za3, Za4, and X are removed from the compound shown by the general formula (iv), and a photosensitizing group is bonded to the nitrogen atoms to which this protective group was bonded.
Step 1-6: The solid phase resin or solid phase compound is separated from the compound obtained in the above step 1-5, a hydroxyl group or an amino group is bonded to the carbon atom with which this solid phase resin or solid phase compound was bonded, the protective groups which were not removed in the step 1-5 are removed, a hydrogen atom is bonded to the nitrogen atom to which this protective group was bonded, and the compound shown by the general formula (1) is thereby obtained.

7. A photoelectrode comprising a conductive substrate, and a porous semiconductor layer formed on this substrate on which the compound shown by the general formula (1) is adsorbed.

8. The dye-sensitized solar cell comprising a photoelectrode of claim 7, and a counter-electrode that opposes to the photoelectrode with an electrolyte layer therebetween.

9. The dye-sensitized solar cell module having two or more of the dye-sensitized solar cells according to claim 8 connected in series as a unit cell.
